# EUROPEAN PATENT SPECIFICATION

(11) **EP 3 787 893 B1**
(45) Date of publication and mention of the grant of the patent: **11.11.2026**
(21) Application number: 19796944.7
(22) Date of filing: 07.05.2019
(51) Int. Cl.: B32B 5/02, B32B 5/14, B33Y 80/00, D03D 1/00, B32B 3/00, B33Y 10/00, B29C 64/10

(54) **SMART COMPOSITE TEXTILES AND METHODS OF FORMING**
INTELLIGENTE VERBUNDTEXTILIEN UND HERSTELLUNGSVERFAHREN
TEXTILES COMPOSITES INTELLIGENTS ET PROCÉDÉS DE FORMATION

(30) Priority: 04.05.2018 US 201862667099 P
(43) Date of publication of application: 10.03.2021
(73) Proprietor: University of New South Wales, Sidney, New South Wales 2052 (AU)
(72) Inventor: KNOTHE TATE, Melissa, UNSW Sidney, New South Wales 2052 (AU)
(74) Representative: Maiwald GmbH
(86) International application number: PCT/IB2019/053708
(87) International publication number: WO 2019/211822

(56) References cited:
- WO-A1-2015/021503
- WO-A1-2015/069619
- WO-A1-2016/191347
- WO-A1-2016/203409
- WO-A1-2019/108794
- CN-A- 103 143 062
- DE-A1- 102016 117 293
- DE-A1- 102016 117 293
- US-A1- 2004 187 714
- US-A1- 2010 331 998
- US-A1- 2016 159 033
- US-A1- 2017 151 733
- US-A1- 2017 151 733
- NG JOANNA L. ET AL: "Scale-up of nature’s tissue weaving algorithms to engineer advanced functional materials", vol. 7, no. 1, 1 March 2017 (2017-03-01), XP055853421, Retrieved from the Internet <URL:https://www.nature.com/articles/srep40396.pdf> DOI: 10.1038/srep40396
- SIDLER HANS JÖRG ET AL: "Prospective Design, Rapid Prototyping, and Testing of Smart Dressings, Drug Delivery Patches, and Replacement Body Parts Using Microscopy Aided Design and ManufacturE (MADAME)", FRONTIERS IN MEDICINE, vol. 5, 13 December 2018 (2018-12-13), XP055853419, DOI: 10.3389/fmed.2018.00348

## Description

### BACKGROUND

It is desired to provide smarter materials for apparel, architecture, product design and manufacturing, aerospace and automotive industries. However, these capabilities have often required expensive, error-prone and complex electromechanical devices (*e.g.,* motors, sensors, electronics), bulky components, power consumption (*e.g.,* batteries or electricity) and difficult assembly processes. These constraints have made it challenging to efficiently produce dynamic systems, higher-performing machines and more adaptive products.

Further, while "smart" materials have been developed, which can provide some sort of a dynamic structure, such materials are often formed in fixed shapes and sizes. These materials must subsequently be assembled into the necessary end product form, typically using off the shelf (non-custom) parameters. These types of smart materials are extremely expensive and are generally only found in niche markets due to their cost. Further, using these smart materials to provide a specific type of product having a particular function requires significant skill and time.

DE 102016 117 293 A1 describes a composite textile that includes a textile material produced by traditional textile manufacturing and an added pattern of a further material applied by using an additive technique, wherein the textile material has an anisotropic structure at least in sections, wherein the composite textile is set up in such a way that a predictable change in the composite textile occurs in response to an external stimulus using the anisotropic structure of the textile material.

WO 2016/203409 A1 describes a method of forming a substrate comprising: mapping a three dimensional spatial distribution of at least one structural protein fiber and/or natural or synthetic analogs thereof of extracellular matrix of biological material of interest; designing a fiber assembly pattern based on an intrinsic pattern of the at least one structural protein fiber and/or natural or synthetic analogs thereof of the extracellular matrix of the biological material; and assembling fibers based on the fiber assembly pattern to form the substrate.

### SUMMARY

Embodiments described herein relate to engineered composite textiles and/or smart materials formed therefrom as well as to methods of forming the composite textiles and/or smart materials.

In one embodiment, the present invention is directed to a smart material comprising: a composite textile that includes a textile substrate formed from a plurality of fibers assembled in a fiber assembly pattern; and a material deposited via an additive manufacturing technique onto and/or between the fibers of the textile substrate based on an additive manufacturing pattern, wherein the composite textile includes a gradient in least one of mechanical property, material property, or structural property and/or exhibits a change in at least one mechanical property, material property, or structure in response to at least one external stimulus, wherein the additive manufacturing pattern is based on a three dimensional spatial distribution of pores in a biological material of interest and the deposited material defines a matrix that includes a plurality of pores with a hierarchical porosity and/or porosity pattern in the composite textile.

The engineered materials can replicate or mimic biological or natural material's or nature's intrinsic architecture of structural molecules, such as proteins, by translation of nature's intrinsic architecture to weave scaled-up, multidimensional composite textile architectures emulating natural material organization. The methods and composite textiles described herein can provide mechanically functional textiles, including but not limited to engineered tissue fabrics and tissue implants, and materials for transport and safety industries, biomedical materials, absorbent articles, drug delivery devices, bioprosthetic devices, biomaterial implants, flooring, safety devices, and/or microfluidic devices.

In another embodiment, the present invention is directed to a method of forming a smart composite textile, like an engineered smart composite textile, such as a biomedical material, tissue implant, or mechanically functional textile, the method comprising: mapping a three dimensional spatial distribution of pores of a biological material of interest; designing an additive manufacturing pattern based on the three dimensional spatial distribution of pores of the biological material of interest; assembling a plurality of fibers based on a fiber assembly pattern into a textile substrate; depositing a material via an additive manufacturing technique onto and/or between fibers of the textile substrate based on the additive manufacturing pattern to provide a composite textile that includes a gradient in least one of mechanical property, material property, or structural property and/or that exhibits a change in at least one mechanical property, material property, or structure in response to at least one external stimulus; wherein the additive manufacturing pattern is based on a three dimensional spatial distribution of pores in a biological material of interest and the deposited material defines a matrix that includes a plurality of pores with a hierarchical porosity and/or porosity pattern in the composite textile.

In some embodiments, the method can further include mapping a three dimensional spatial distribution of at least one mechanical property, material property, or structure of a natural or biological material of interest. The fiber assembly pattern and/or the additive manufacturing pattern can then be designed based on the intrinsic pattern of the at least one mechanical property, material property, or structural property of the natural or biological material of interest. For example, the fiber assembly pattern can be designed based on an intrinsic pattern of at least one structural molecule of a natural or biological material. The fibers can then be assembled based on the fiber assembly pattern to form the textile substrate.

The structural molecule can include at least one structural protein fiber of the extracellular matrix. The at least one structural protein fiber can include collagen fibers and elastin fibers of the extracellular matrix of the biological material, such as a plant or animal.

In some embodiments, the fiber assembly pattern can include a weaving algorithm based on the intrinsic pattern. The assembled fibers can be woven using the weaving algorithm to define the weave pattern and fiber orientation.

In other embodiments, the additive manufacturing technique can include one or more of a fused deposition modeling (FDM) technique, a fused filament fabrication (FFF) technique, a big area additive manufacturing (BAAM) technique, a robocasting technique, a paste extrusion technique, an electrospinning technique a direct ink writing (DIW) technique, or 3D printing technique.

As indicated above, the deposited material defines a matrix that includes a plurality of pores with a hierarchal porosity and/or porosity pattern in the composite textile. Furthermore, the additive manufacturing pattern for the deposited material is based on a three dimensional spatial distribution of pores in a biological material of interest.

In other embodiments, a fluid can be provided within the pores. The movement of the fluid in the pores can dissipate energy in response to force or impact on and/or of the composite textile.

In some embodiments, the pores can have a hierarchy and/or gradient such that composite textile includes a first region that exudes fluid in response to a compressive or tensile load and a second region that imbibes fluid in response to the load. The first region and the second region can extend from an outer surface of the composite textile. In response to compressive or tensile load to the composite textile, the first region can exude fluid from the outer surface toward the direction of the load, and the second region can imbibe fluid from the outer surface away from the direction of the load.

In other embodiments, the first region can include a first fluid. The first fluid can flow from the first region in response to compressive or tensile load. In some embodiments, the first region can include a first porous material having a first porosity and the second region comprising a second porous material having a second porosity different that the first porosity.

In other embodiments, the composite textile can include a plurality of first regions laterally spaced from one another in the composite textile and separated by the second region. At least some of the first regions can have a different porosity, volume, volumetric permeability, and/or surface permeability than the porosity, volume, volumetric permeability, and/or surface permeability of other first regions.

In other embodiments, the composite textile can have a region of temporally-controlled elasticity that transitions between a first state and a second state in response to the external stimuli. The first state can be more relaxed than the second state, and the smart material can at least partially revert from the second state to the first state over an extended time period resulting from the temporally-controlled elasticity of the textile substrate. The internal energy of the smart material in the first state can be less than internal energy of the substrate in the second state. Different regions of the smart material can possess different temporally-controlled elasticity.

In some embodiments, the smart material can move from the second state to the first state via any one of elongation or shortening of the smart material, or relaxation or stiffening of the smart material. The textile substrate can possess spatially-controlled elasticity, whereby different regions of the textile substrate have different elasticity or stiffness.

In some embodiments, the textile substrate can be woven using at least two threads/fibers, wherein each thread has a different elasticity.

In other embodiments, the textile substrate can include at least one thread possessing elasticity that varies along the length of the thread. The textile substrate can include at least one thread possessing elasticity that varies within the cross-section of the thread.

In other embodiments, the textile substrate can be woven using threads arranged in different directions such that the threads move frictionally relative to one another causing the transition from the first state to the second state to occur over an extended time period.

In some embodiments, the smart material or composite textile can further include at least one bioactive agent incorporated on or within the composite textile. The at least one bioactive agent can be capable modulating a function and/or characteristic of a cell. The bioactive material can include, for example, chemotactic agents, various proteins *(e.g.,* short term peptides, bone morphogenic proteins, collagen, glycoproteins, and lipoprotein), cell attachment mediators, biologically active ligands, integrin binding sequence, various growth and/or differentiation agents and fragments thereof (*e.g.,* epidermal growth factor (EGF), hepatocyte growth factor (HGF), vascular endothelial growth factors (VEGF), fibroblast growth factors (*e.g*., bFGF), platelet derived growth factors (PDGF), insulin-like growth factor *(e.g.,* IGF-I, IGF-II) and transforming growth factors (*e.g.,* TGF-β I-III)), parathyroid hormone, parathyroid hormone related peptide, bone morphogenic proteins (*e.g.,* BMP-2, BMP-4, BMP-6, BMP-7, BMP-12, BMP-13, BMP-14), transcription factors, such as sonic hedgehog, growth differentiation factors (*e.g.,* GDF5, GDF6, GDF8), recombinant human growth factors (*e.g.,* MP52 and the MP-52 variant rhGDF-5), cartilage-derived morphogenic proteins (CDMP-1, CDMP-2, CDMP-3), small molecules that affect the upregulation of specific growth factors, tenascin-C, hyaluronic acid, chondroitin sulfate, fibronectin, decorin, thromboelastin, thrombin-derived peptides, heparin-binding domains, heparin, heparan sulfate, polynucleotides, DNA fragments, DNA plasmids, MMPs, TIMPs, interfering RNA molecules, such as siRNAs, oligonucleotides, proteoglycans, glycoproteins, glycosaminoglycans, and DNA encoding for shRNA.

In other embodiments, the smart material or composite textile can include at least one cell dispersed on and/or within the composite textile. The cell can be, for example, a progenitor cell, such as totipotent stem cells, pluripotent stem cells, and multipotent stem cells, as well as any of their lineage descendant cells, including more differentiated cells. Exemplary progenitor cells can be selected from, but not restricted to, totipotent stem cells, pluripotent stem cells, multipotent stem cells, mesenchymal stem cells (MSCs), hematopoietic stem cells, neuronal stem cells, hematopoietic stem cells, pancreatic stem cells, cardiac stem cells, embryonic stem cells, embryonic germ cells, neural crest stem cells, kidney stem cells, hepatic stem cells, lung stem cells, hemangioblast cells, and endothelial progenitor cells. Additional exemplary progenitor cells are selected from, but not restricted to, de-differentiated chondrogenic cells, chondrogenic cells, cord blood stem cells, multipotent adult progenitor cells, myogenic cells, osteogenic cells, tendogenic cells, ligamentogenic cells, adipogenic cells, and dermatogenic cells.

Still other embodiments relate to a wound dressing that includes a composite textile. The composite textile includes a textile substrate formed from a plurality of fibers assembled in a fiber assembly pattern and a material deposited via an additive manufacturing technique onto and/or between the fibers of the textile substrate based on an additive manufacturing pattern. The deposited material can define a matrix that includes plurality of pores with a hierarchal porosity and/or porosity gradient and/or porosity pattern in the composite textile such that composite textile includes a first region that exudes fluid in response to a compressive or tensile load and a second region that imbibes fluid in response to the load.

Other embodiments relate to armor, such as body armor, that includes a composite textile. The composite textile can include a textile substrate formed from a plurality of fibers assembled in a fiber assembly pattern and a material deposited via an additive manufacturing technique onto and/or between the fibers of the textile substrate based on an additive manufacturing pattern. The deposited material defines a matrix that includes plurality of pores with a hierarchal porosity and/or porosity gradient and/or porosity pattern in the composite textile. A fluid, such as a liquid, is provided within the pores. The movement of the fluid in the pores can dissipate energy in response to force impact on or of the composite textile.

### BRIEF DESCRIPTION OF THE DRAWINGS

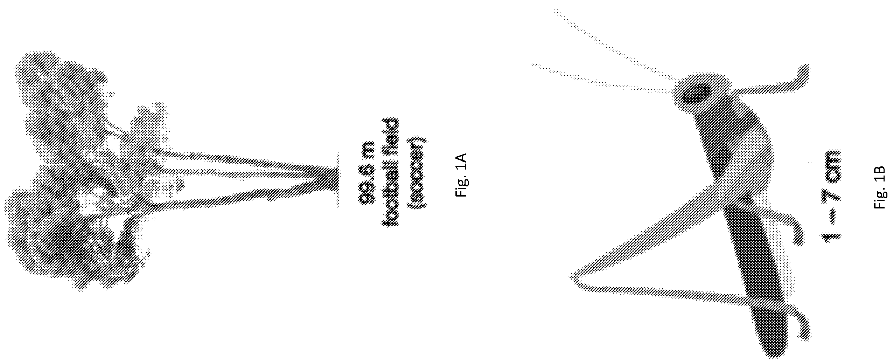

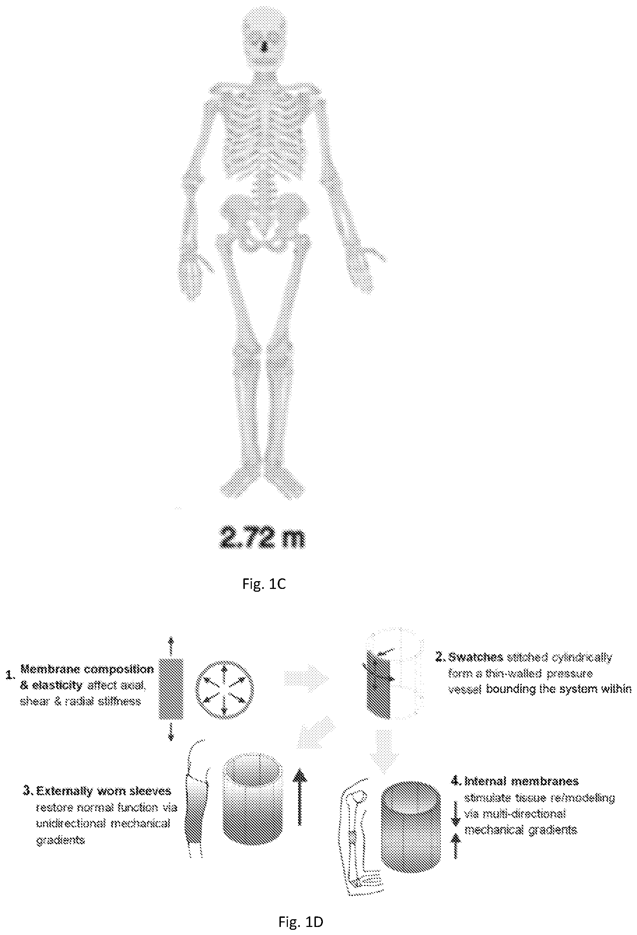

Figs. 1(A-D) illustrate a schematic showing a design and manufacturing process that is applicable for the creation of diverse materials exhibiting unique gradients in mechanical structure. These gradients underpin the remarkable higher order function of such structures. For example, (A) the towering eucalyptus tree that bends like a blade of grass in high winds, (B) the mechanical gradients intrinsic to joint function in insect exoskeletons, and (C) the internal musculoskeletal system of vertebrates are all enabled through prescient distribution of mechanical properties in space and time. Nature provides infinite patterns that provide inspiration for ideation of smart materials. (D) Such mechanical gradient properties can be implemented to harness natural movements (D1, D2) for external (wearables, D3) and internal (implants, D4) applications that harness the movement of the local system *e.g.,* to deliver directional pressure gradients and/or gradients in strain at interfaces.

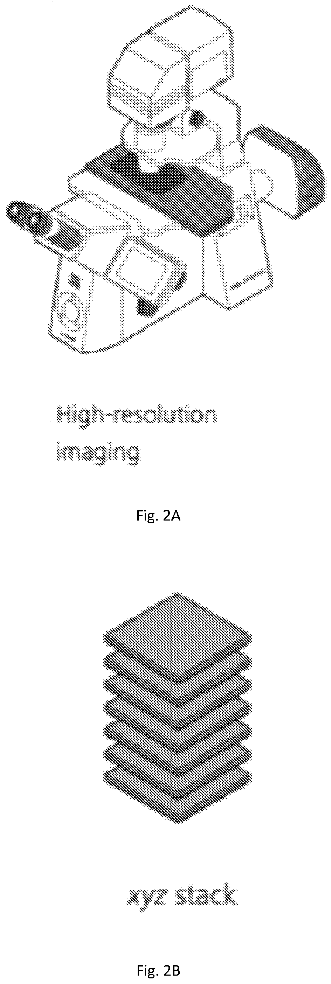

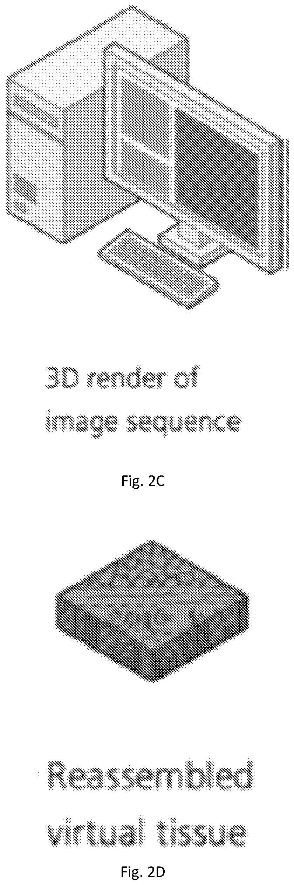

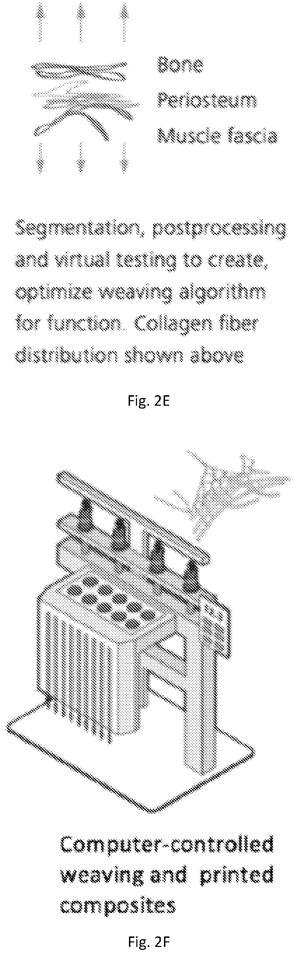

Figs. 2(A-F) illustrate a schematic showing a process for microscopy-enabled, scaled-up computer-aided design, and manufacture of composite multifunctional textiles and 3D prints emulating the body's own tissues. (A-D) Second harmonic generation and two photon microscopy of tissues reveals a spatial map of elastin and collagen, *e.g.,* in the periosteum, a soft, and elastic tissue sheath that bounds all non-articular surfaces of bone. In this example, microscopy is used to map the precise pattern of elastin and collagen in native tissue. As the initial step in the pipeline, the raw microscopy data is thus transformed to patterns of representing material properties, *e.g.,* stiffness. (E) These tissue maps are then rendered using computer-aided design software, where the patterns can be optimized for desired design specifications. This step in the pipeline creates stl files that are input into rapid manufacturing processes including *e.g.,* integrated weaving and/or multi-dimensional printing. (F) Optimized designs thus provide inputs for computer controlled weaving of textiles and combined printing of composites that emulate the tissue studied under the microscope.

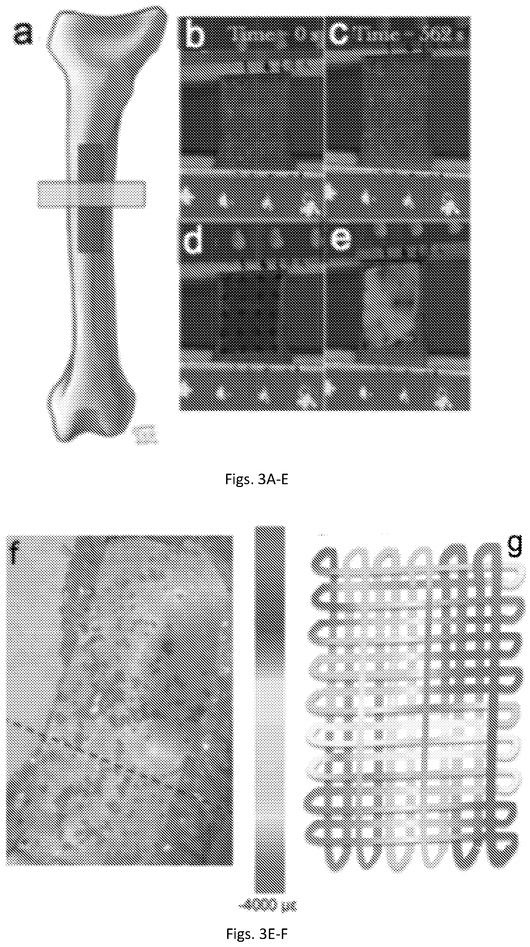

Figs. 3(A-G) illustrate recursive weaving of advanced materials that emulate Nature's own. (A-E) Example depicting anisotropic mechanical properties of periosteum, the hyperelastic sheath covering all bony surfaces in vertebrates. In the sheep femur (A) strain maps are created during loading in tension using digital image correlation, on sections of periosteum (A-E) cut in either the longitudinal or circumferential direction (A). High resolution strain maps of the entire periosteum of the femur, *in situ* during stance shift loading, show heterogeneity of mechanical properties in space and time over the course of the loading cycle [(F), still image taken from single frame of digital video over the loading cycle]. (G) Conceptually, a singular solution to recursively weave the tissue fabric of the periosteum tested would be to "unravel" a single strand's mechanical properties that would vary along the entire length of the strand. Many more solutions exist through creation of fiber patterns comprised of elastic and tough fibers such as elastin and collagen using computer-controlled weaving.

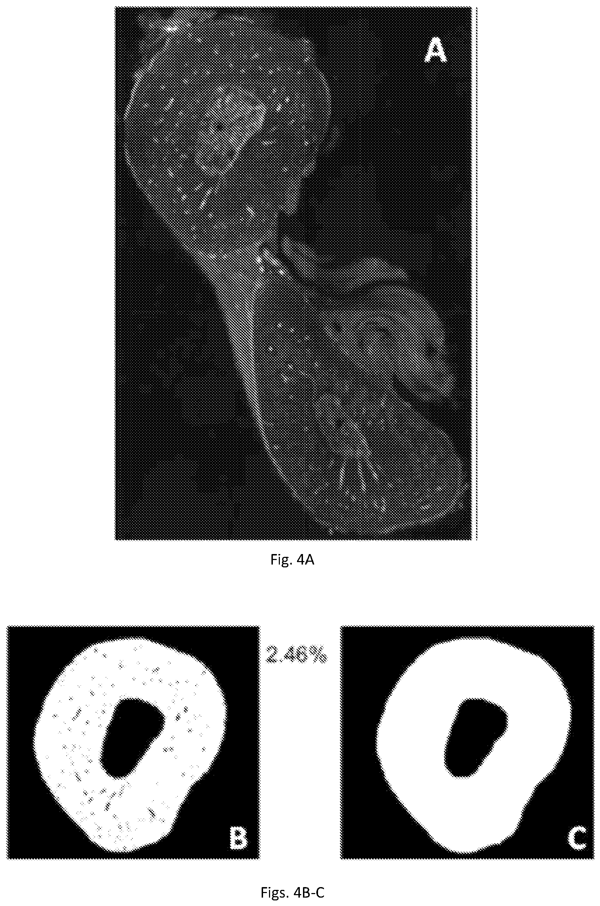

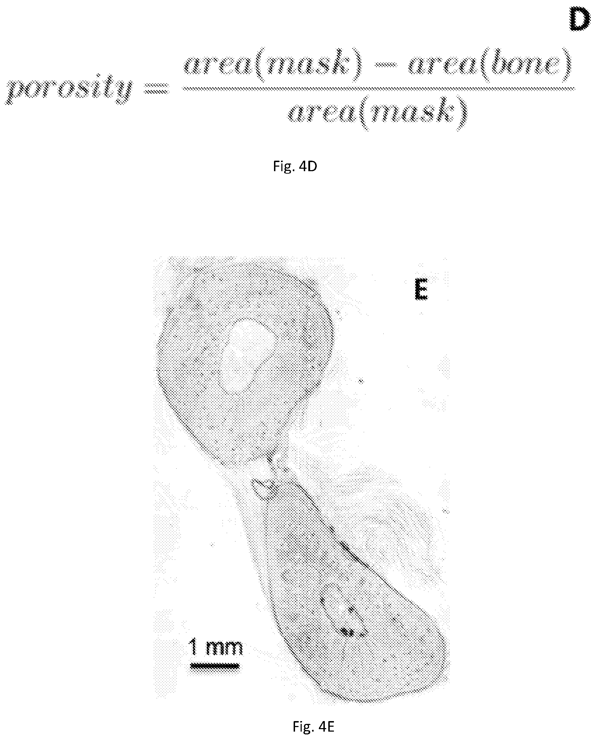

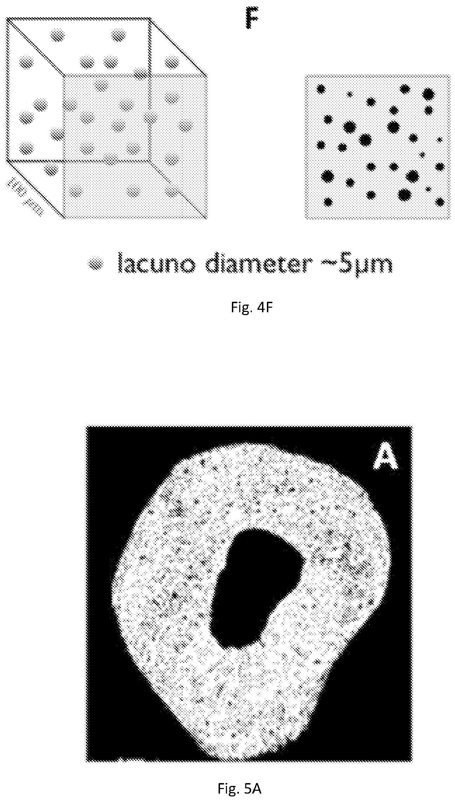

Figs. 4(A-F) illustrate mapping of the vascular porosity in bone. (A) Fluorescent confocal image. (B) Mask depicting area with vascular pores, *area*(*bone*) in the equation (D). (C) Mask depicting area without vascular pores or *area*(*mask*) in the equation (D). (D) Equation to calculate vascular porosity. (E,F) Calculation of lacunar porosity in bone, using (E) transmitted light images.

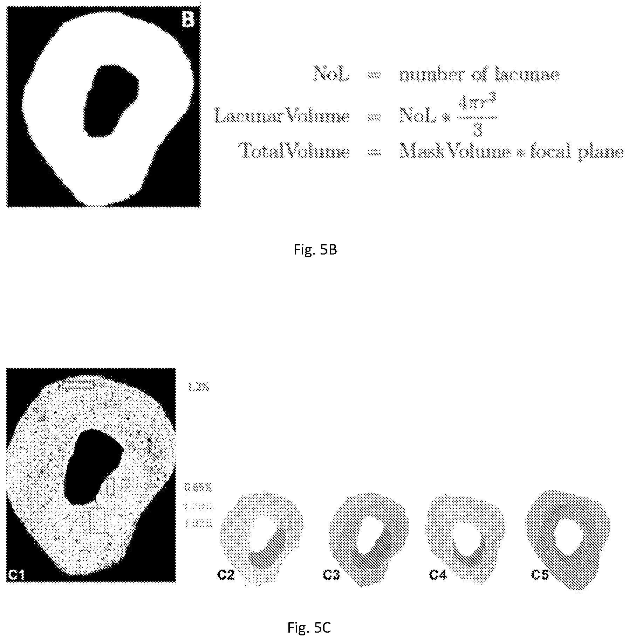

Figs. 5(A-C) illustrate mapping of the lacunar porosity in bone using transmitted light images (A,B) and mapping of site specific lacunar porosity in bone (C1-5). (A) Mask of bone with lacunae. (B) MaskVolume of bone without lacunae. Based on the calculations, the lacunar porosity is 1.1% for the example shown.

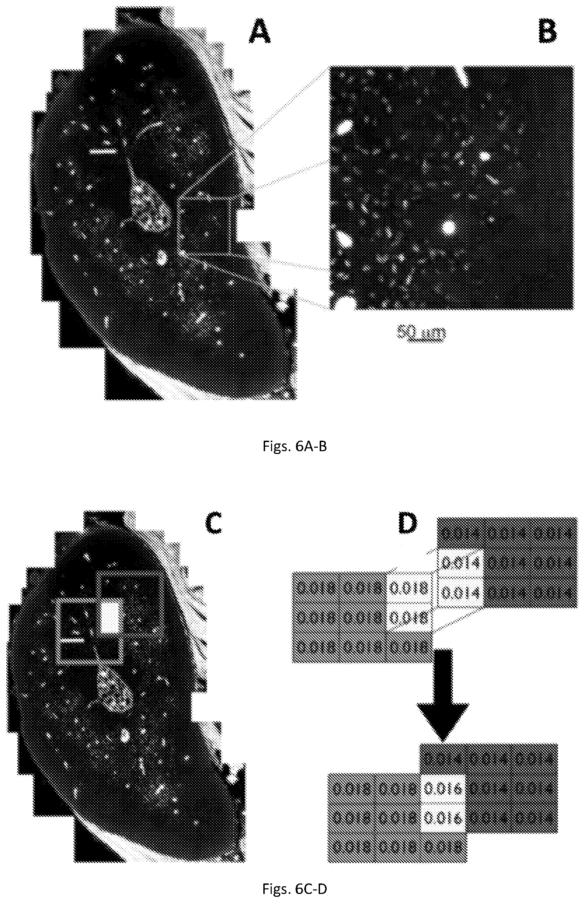

Figs. 6(A-D) illustrate from high resolution maps of different caliber porosities [vascular, lacunar-(A,B)] to generation of matrices representing imaging data (C,D).

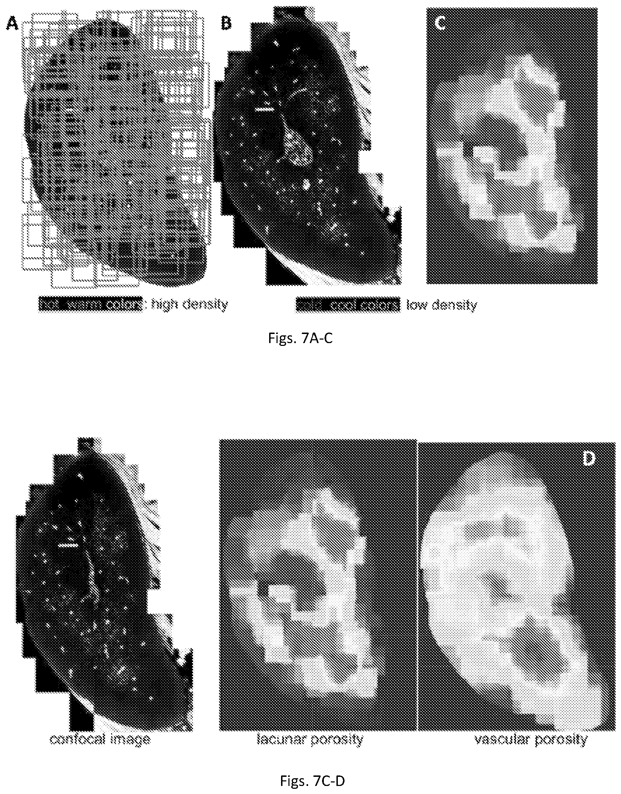

Figs. 7(A-D) illustrate heat maps are generated from random assessment of areas (A), for lacunar and vascular porosity (B) in this case, and depicted as density gradients (C,D), using hot-warm colors and low density using cool colors.

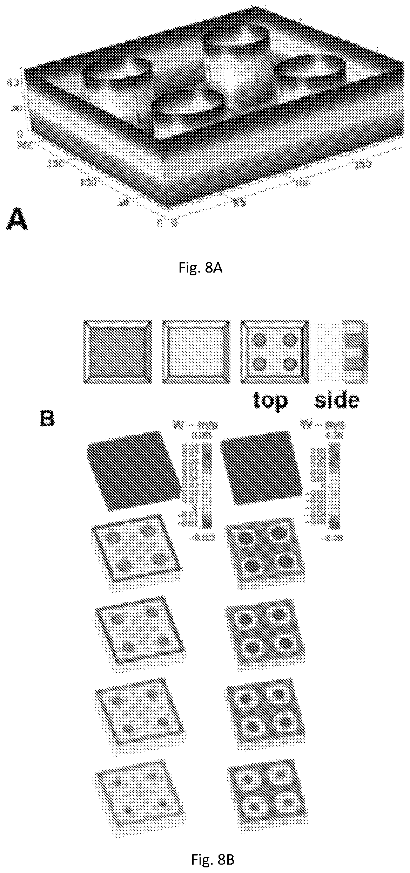

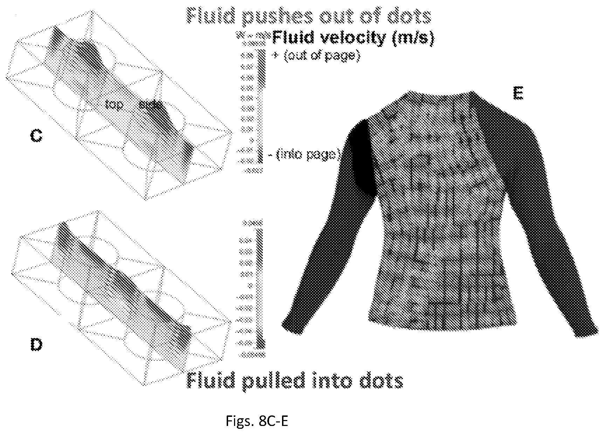

Figs. 8(A-E) illustrate application of MADAME to designer dressings and wearables. Modular designs (A) can be scaled up and tuned *e.g.,* for bespoke bandages with spatial and temporal control of drug delivery. (B-D) Directionality of delivery dots and surrounding areas can be controlled by the architecture of the module. Scale bars depict fluid velocity, with warm colors indicating flow outwards and cool colors, flow inwards; *e.g.,* pushing on the patch (B,C) results in flow out of the delivery dots. (E) Example of large scale, wearable wound dressing for *e.g.,* burn treatment.

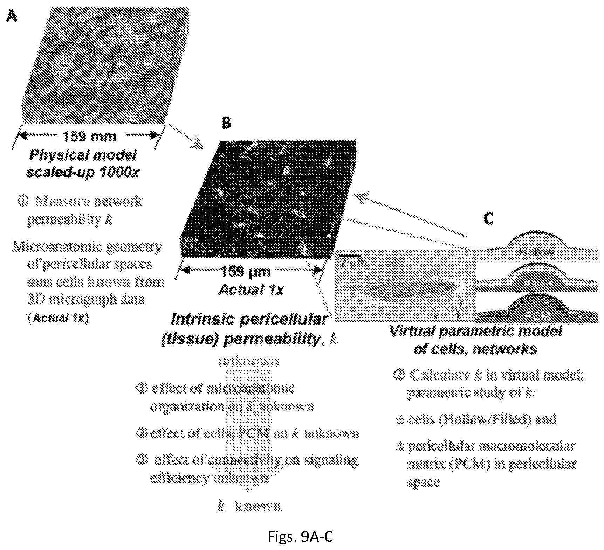

Figs. 9(A-C) illustrate early example of scale up and rapid prototyping of micron scale systems to emulate smart permeability properties in 1,000x scaled up (cm length scale) system. The intrinsic tissue permeability cannot be measured based on microscopy alone (B). 1,000x scaled up physical renderings of the microscopic data are depicted as inverse microscopy data to encode flow around cells and their networks (A). Virtual renderings of single cells enable analysis of the effect of pericellular matrix permeability on bulk pericellular tissue permeability (C). Only through parallel study of virtual, scaled up physical renderings, and virtual *in silico* modeling based renderings of the system at different length scales, can the interactions between the elements and bulk properties of the tissue be estimated and validated. These studies were the first of their kind and they paved the way for organ to nano scale maps of human tissues and organs using other imaging modalities.

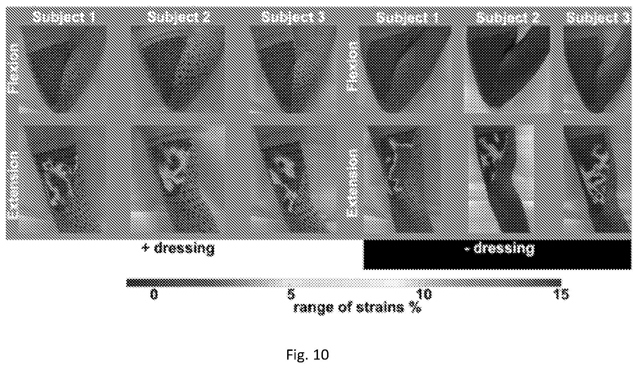

Fig. 10 illustrates coupled experimental mechanics and modeling studies enable determination of the range of strains on the surface of the human arm typical for daily activities. Digital imaging correlation methods and custom computer code developed for mapping strains *in situ* on the surface of the periosteum (Fig. 3) were used to measure strain on the surface of the arms of three subjects, with and without the presence of a compressive dressing. Strains are mapped at one point in time (one frame of digital video) during flexion and compression of the arm.

### DETAILED DESCRIPTION

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter.

Following long-standing patent law tradition, the terms "a", "an", and "the" are meant to refer to one or more as used herein, including the claims. For example, the phrase "a cell" can refer to one or more cells.

The term "absorbable" is meant to refer to a material that tends to be absorbed by a biological system into which it is implanted. Representative absorbable fiber materials include, but are not limited to polyglycolic acid (PGA), polylactic acid (PLA), polyglycolide-lactide, polycaprolactone, polydioxanone, polyoxalate, a polyanhydride, a poly(phosphoester), catgut suture, collagen, silk, chitin, chitosan, hydroxyapatite, bioabsorbable calcium phosphate, hyaluronic acid, and any other medically acceptable yet absorbable fiber. Other absorbable materials include collagen, gelatin, a blood derivative, plasma, synovial fluid, serum, fibrin, hyaluronic acid, a proteoglycan, elastin, and combinations thereof.

The term "non-absorbable" is meant to refer to a material that tends not to be absorbed by a biological system into which it is implanted. Representative non-absorbable fiber materials include but are not limited to polypropylene, polyester, polytetrafluoroethylene (PTFE) such as that sold under the registered trademark TEFLON (E.I. DuPont de Nemours & Co., Wilmington, Del., United States of America), expanded PTFE (ePTFE), polyethylene, polyurethane, polyamide, nylon, polyetheretherketone (PEEK), polysulfone, a cellulosic, fiberglass, an acrylic, tantalum, polyvinyl alcohol, carbon, ceramic, a metal (*e.g.,* titanium, stainless steel), and any other medically acceptable yet non-absorbable fiber.

The terms "anisotropic", "anisotropy", and grammatical variations thereof, refer to properties of a textile, composite, and/or fiber system as disclosed herein that can vary along a particular direction. Thus, the fiber, composite, and/or textile can be stronger and/or stiffer in one direction versus another. In some embodiments, this can be accomplished by changing fibers (such as, but not limited to providing fibers of different materials) in warp versus weft directions, and/or in the Z direction, for example, or changing the material disposed using the additive manufacturing technique.

The terms "anisotropic", "anisotropy" and grammatical variations thereof, can also include, but is not limited to the provision of more fiber or disposed material in a predetermined direction. This can thus include a change of diameter in a fiber over a length of the fiber, a change in diameter at each end of the fiber, and/or a change in diameter at any point or section of the fiber; a change in cross-sectional shape of the fiber; a change in density or number of fibers in a volumetric section of the scaffold; the use of monofilament fibers and/or multifilament fibers in a volumetric section of the textile, or the use of different types, amounts, or densities of deposited materials; and can even include the variation in material from fiber system to fiber system and along individual fibers in a volumetric section of the textile.

The terms "biocompatible" and "medically acceptable" are used synonymously herein and are meant to refer to a material that is compatible with a biological system, such as that of a subject having a tissue to be repaired, restored, and/or replaced. Thus, the term "biocompatible" is meant to refer to a material that can be implanted internally in a subject as described herein.

The term "composite material", as used herein, is meant to refer to any material comprising two or more components.

The term "bioactive agent" can refer to any agent capable of promoting tissue formation, destruction, and/or targeting a specific disease state (*e.g.,* cancer). Examples of bioactive agents can include, but are not limited to, chemotactic agents, various proteins *(e.g.,* short term peptides, bone morphogenic proteins, collagen, glycoproteins, and lipoprotein), cell attachment mediators, biologically active ligands, integrin binding sequence, various growth and/or differentiation agents and fragments thereof (*e.g.,* epidermal growth factor (EGF), hepatocyte growth factor (HGF), vascular endothelial growth factors (VEGF), fibroblast growth factors (*e.g.,* bFGF), platelet derived growth factors (PDGF), insulin-like growth factor *(e.g.,* IGF-I, IGF-II) and transforming growth factors *(e.g.,* TGF-β I-III)), parathyroid hormone, parathyroid hormone related peptide, bone morphogenic proteins (*e.g.,* BMP-2, BMP-4, BMP-6, BMP-7, BMP-12, BMP-13, BMP-14), transcription factors, such as sonic hedgehog, growth differentiation factors *(e.g.,* GDF5, GDF6, GDF8), recombinant human growth factors (*e.g.,* MP52 and the MP-52 variant rhGDF-5), cartilage-derived morphogenic proteins (CDMP-1, CDMP-2, CDMP-3), small molecules that affect the upregulation of specific growth factors, tenascin-C, hyaluronic acid, chondroitin sulfate, fibronectin, decorin, thromboelastin, thrombin-derived peptides, heparin-binding domains, heparin, heparan sulfate, polynucleotides, DNA fragments, DNA plasmids, MMPs, TIMPs, interfering RNA molecules, such as siRNAs, oligonucleotides, proteoglycans, glycoproteins, glycosaminoglycans, and DNA encoding for shRNA.

The term "bioresorbable" can refer to the ability of a material to be fully resorbed *in vivo.* "Full" can mean that no significant extracellular fragments remain. The resorption process can involve elimination of the original implant material(s) through the action of body fluids, enzymes, cells, and the like.

The term "cell" can refer to any progenitor cell, such as totipotent stem cells, pluripotent stem cells, and multipotent stem cells, as well as any of their lineage descendant cells, including more differentiated cells. The terms "stem cell" and "progenitor cell" are used interchangeably herein. The cells can derive from embryonic, fetal, or adult tissues. Exemplary progenitor cells can be selected from, but not restricted to, totipotent stem cells, pluripotent stem cells, multipotent stem cells, mesenchymal stem cells (MSCs), hematopoietic stem cells, neuronal stem cells, hematopoietic stem cells, pancreatic stem cells, cardiac stem cells, embryonic stem cells, embryonic germ cells, neural crest stem cells, kidney stem cells, hepatic stem cells, lung stem cells, hemangioblast cells, and endothelial progenitor cells. Additional exemplary progenitor cells are selected from, but not restricted to, de-differentiated chondrogenic cells, chondrogenic cells, cord blood stem cells, multipotent adult progenitor cells, myogenic cells, osteogenic cells, tendogenic cells, ligamentogenic cells, adipogenic cells, and dermatogenic cells.

The term "effective amount" refers to an amount of a bioactive agent sufficient to produce a measurable response *(e.g.,* a biologically relevant response in a cell exposed to the differentiation-inducing agent) in the cell. In some embodiments, an effective amount of a differentiation-inducing agent is an amount sufficient to cause a precursor cell to differentiate in *in vitro* culture into a cell of a tissue at predetermined site of treatment. It is understood that an "effective amount" can vary depending on various conditions including, but not limited to the stage of differentiation of the precursor cell, the origin of the precursor cell, and the culture conditions.

The terms "inhomogeneous", "inhomogeneity", "heterogeneous", "heterogeneity", and grammatical variations thereof, are meant to refer to a fiber, substrate, textile, composite, and/or fabric as disclosed herein that does not have a homogeneous composition along a given length or in a given volumetric section. In some embodiments, an inhomogeneous construct as disclosed herein comprises a composite material, such as a composite comprising a three dimensional woven fiber substrate, textile, and/or fabric as disclosed herein, cells that can develop tissues that substantially provide the function of periosteum, cartilage, other tissues, or combinations thereof, and a matrix that supports the cells. In some embodiments, an inhomogeneous substrate as disclosed herein can comprise one or more component systems that vary in their properties according to a predetermined profile, such as a profile associated with the tissue and/or other location in a subject where the substrate will be implanted. Thus, it is an aspect of the terms "inhomogeneous", "inhomogeneity", "heterogeneous", "heterogeneity", and grammatical variations thereof to encompass the control of individual materials and properties in the substrate.

The terms "non-linear", "non-linearity", and grammatical variations thereof, refer to a characteristic provided by a fiber substrate, textile, and/or fabric as disclosed herein such that the fiber substrate, textile, and/or fabric can vary in response to a strain. Fiber substrate, textile, and/or fabric disclosed herein can provide stress/stain profiles that mimic that observed in a target or region of interest.

The terms "resin", "matrix", or "gel" are used in the art-recognized sense and refer to any natural or synthetic solid, liquid, and/or colloidal material that has characteristics suitable for use in accordance with the presently disclosed subject matter. Representative "resin", "matrix", or "gel" materials thus comprise biocompatible materials. In some embodiments, the "resin", "matrix", or "gel" can occupy the pore space of a textile substrate as disclosed herein.

The term "smart material(s)" refers to a designed material that have one or more properties that can be changed in a controlled fashion under the influence of an external stimulus, such as stress, temperature, moisture, pH, electric or magnetic fields. This change can be reversible and can be repeated many times.

As used herein, "structural material" means a material used in constructing a wearable, personal accessory, luggage, etc. Examples of structural materials include: fabrics and textiles, such as cotton, silk, wool, nylon, rayon, synthetics, flannel, linen, polyester, woven or blends of such fabrics, etc.; leather; suede; pliable metallic such as foil; Kevlar, etc. Examples of wearables include: clothing; footwear; prosthetics such as artificial limbs; headwear such as hats and helmets; athletic equipment worn on the body; protective equipment such as ballistic vests, helmets, and other body armor. Personal accessories include: eyeglasses; neckties and scarfs; belts and suspenders; jewelry such as bracelets, necklaces, and watches (including watch bands and straps); and wallets, billfolds, luggage tags, etc. Luggage includes: handbags, purses, travel bags, suitcases, backpacks, and including handles for such articles, etc.

The terms "viscoelastic", "viscoelasticity", and grammatical variations thereof, are meant to refer to a characteristic provided by a fiber substrate, textile, and/or fabric as disclosed herein that can vary with a time and/or rate of loading.

In one embodiment, the present invention is directed to a smart material comprising: a composite textile that includes a textile substrate formed from a plurality of fibers assembled in a fiber assembly pattern; and a material deposited via an additive manufacturing technique onto and/or between the fibers of the textile substrate based on an additive manufacturing pattern, wherein the composite textile includes a gradient in least one of mechanical property, material property, or structural property and/or exhibits a change in at least one mechanical property, material property, or structure in response to at least one external stimulus, wherein the additive manufacturing pattern is based on a three dimensional spatial distribution of pores in a biological material of interest and the deposited material defines a matrix that includes a plurality of pores with a hierarchical porosity and/or porosity pattern in the composite textile.

In some embodiments, the engineered composite textiles can replicate or mimic biological or natural material's or nature's intrinsic architecture of structural molecules, such as proteins, by translation of nature's intrinsic architecture to weave scaled-up, multidimensional composite textile architectures emulating natural material organization. The methods and composite textiles described herein can provide mechanically functional textiles, including but not limited engineered tissue fabrics and tissue implants, and materials for transport and safety industries, structural material, biomedical materials, absorbent articles, drug delivery devices, bioprosthetic devices, biomaterial implants, flooring, safety devices, and/or microfluidic devices.

In another embodiment, the present invention is directed to a method of forming a smart composite textile, like an engineered smart composite textile, such as a biomedical material, tissue implant, or mechanically functional textile, the method comprising: mapping a three dimensional spatial distribution of pores of a biological material of interest; designing an additive manufacturing pattern based on the three dimensional spatial distribution of pores of the biological material of interest; assembling a plurality of fibers based on a fiber assembly pattern into a textile substrate; depositing a material via an additive manufacturing technique onto and/or between fibers of the textile substrate based on the additive manufacturing pattern to provide a composite textile that includes a gradient in least one of mechanical property, material property, or structural property and/or that exhibits a change in at least one mechanical property, material property, or structure in response to at least one external stimulus; wherein the additive manufacturing pattern is based on a three dimensional spatial distribution of pores in a biological material of interest and the deposited material defines a matrix that includes a plurality of pores with a hierarchical porosity and/or porosity pattern in the composite textile.

In some embodiments, the method can further include mapping a three dimensional spatial distribution of at least one mechanical property, material property, or structure of a natural or biological material of interest. For example, the fiber assembly pattern can be designed based on an intrinsic pattern of at least one structural molecule of a natural material or a biological material. The structural molecule can include, for example, a structural protein fiber, such as collagen fibers, elastin fibers, fibronectin fibers, and laminin fibers. In some examples, the at least one structural protein fiber can include collagen fibers and elastin fibers (and/or natural or synthesized analogs thereof) of the extracellular matrix of the biological material.

In some embodiments, the biological material can include any biological material that comprises an extracellular matrix of structural protein fibers including tissue of a plant or animal. The tissue can include, for example, at least one or periosteum, pericardium, perimycium, or tissue bounding an organ or tissue compartment (e.g., tree bark).

Regions of interest (ROI) of the natural or biological material can be imaged and mapped to highlight gradients of mechanical properties, material properties, or structure of a natural or biological material. For example, ROI in context of tissue compartments (bone, muscle, vasculature) and their respective microscopic structures can be mapped along the major and minor axes. These axes, calculated using an automated software, can serve, for example, as objective indicators of tissue regions most and least able to resist bending forces in the axial plane. For each ROI, a tiled image of the transverse (xy) plane, followed by a z-stack of one tile within the region, can be captured to map in 3D space the composition and distribution of structural molecules, such as collagen and elastin fibers, as well as their higher order architectures.

In some embodiments, the three dimensional spatial distribution of structural protein fibers (*e.g.,* collagen fibers and elastin fiber) can be mapped or imaged using multimodal imaging of section or transverse section of a biological material. For example, the three dimensional spatial distribution of the collagen fibers and the elastin fibers can be mapped using, respectively, second harmonic imaging microscopy and two photon excitation imaging microscopy of transverse section of ROI of the biological material.

Second harmonic imaging microscopy (SHIM) can be used to capture high-resolution, high-content, 3D representations of fibrillar collagen in live and *ex vivo* tissue without the need for exogenous labeling. In SHIM, a frequency doubling of the incident light occurs in repetitive and non-centrosymmetric molecular structures.

By way of example, biological specimens can be imaged using a Leica SP5 II inverted microscope equipped with a Spectra Physics MaiTai HP DeepSea titanium sapphire multiphoton laser tuned to 830 nm (~100 fs pulse), a xyz high precision multipoint positioning stage and a 63x 1.3NA glycerol objective. The forward propagated second harmonic collagen signal can then be collected in the transmitted Non-Descanned-Detector using a 390-440 nm bandpass filter.

The two-photon imaging of elastin can be performed by excitation of the biological specimen at 830 nm and following by collection using a photo-multiplier tube (PMT) with a 435-495 nm emission filter. This filter can be used to segment away autofluorescence.

The images can then collated to create to create scaled up three dimensional maps or models, which accurately represent the composition and spatial architecture of the image sequences and the extracellular matrix, biological material, and/or tissue itself. The three dimensional maps can include not only the spatial distribution of the structural molecules, such as collagen fibers and elastin fibers, but also other features or structures including vasculature that extends through the matrix.

Following mapping of the three dimensional spatial distribution of at least one mechanical property, material property, or structure of a natural or biological material, such as collagen fiber and elastin fiber of the extracellular matrix, a fiber assembly pattern can be designed based on an intrinsic pattern of the mapped mechanical property, material property, or structural property. In some embodiments, the fiber assembly pattern can include a weaving algorithm or weaving motif based on the intrinsic pattern of the mapped three dimensional spatial distribution mechanical property, material property, or structural property as well as other structural features. In some instances, the intrinsic pattern of the can be used to design or generate a custom-configured jacquard weaving algorithm (ArahWeave, arahne CAD/CAM for weaving) for weaving of physical prototypes (AVL Looms, Inc.).

Following design of the fiber assembly pattern, fibers are woven in a weave pattern and/or fiber orientation based on the fiber assembly pattern or weaving algorithm to form a textile substrate. The fibers woven using the weaving algorithm can be monofilament, multifilament, or a combination thereof, and can be of any shape or cross-section including, but not limited to bracket-shaped (*i.e.,* [), polygonal, square, I-beam, inverted T shaped, or other suitable shape or cross-section. The cross-section can vary along the length of fiber. Fibers can also be hollow to serve as a carrier for bioactive agents (*e.g.,* antibiotics, growth factors, etc.), cells, and/or other materials as described herein. In some embodiments, the fibers can serve as a degradable or non-degradable carriers to deliver a specific sequence of growth factors, antibiotics, or cytokines, etc., embedded within the fiber material, attached to the fiber surface, or carried within a hollow fiber. The fibers can each comprise a biocompatible material, and the biocompatible material can comprise an absorbable material, a non-absorbable material, or combinations thereof.

Fiber diameters can be of any suitable length in accordance with characteristics composite textile's use or function. Representative size ranges include a diameter of about 1 micron, about 5 microns, about 10 microns about 20 microns, about 40 microns, about 60 microns, about 80 microns, about 100 microns, about 120 microns, about 140 microns, about 160 microns, about 180 microns, about 200 microns, about 220 microns, about 240 microns, about 260 microns, about 280 microns, about 300 microns, about 320 microns, about 340 microns, about 360 microns, about 380 microns, about 400 microns, about 450 microns or about 500 microns (including intermediate lengths). In various embodiments, the diameter of the fibers can be less than about 1 micron or greater than about 500 microns. Additionally, nanofibers fibers with diameters in the nanometer range (1-1000 nanometers) are envisioned for certain embodiments. Additionally, large fibers with diameters up to 3.5 cm are envisioned for certain embodiments.

In other embodiments, the fibers or subset of fibers, can contain one or more bioactive or therapeutic agents such that the concentration of the bioactive or therapeutic agent or agents varies along the longitudinal axis of the fibers or subset of fibers. The concentration of the active agent or agents can vary linearly, exponentially or in any desired fashion, as a function of distance along the longitudinal axis of a fiber. The variation can be monodirectional; that is, the content of one or more therapeutic agents can decrease from the first end of the fibers or subset of the fibers to the second end of the fibers or subset of the fibers. The content can also vary in a bidirectional fashion; that is, the content of the therapeutic agent or agents can increase from the first ends of the fibers or subset of the fibers to a maximum and then decrease towards the second ends of the fibers or subset of the fibers.

Thus, in some embodiments, the fibers serve as a degradable or nondegradable carrier to deliver one or more specific sequences of growth factors, antibiotics, cytokines, etc. that are embedded within the fiber matter, attached to the fiber surface, or carried within a hollow fiber.

In some embodiments, the fibers woven to form the textile substrate can be prepared in a hydrated form or it can be dried or lyophilized into a substantially anhydrous form.

In other embodiments, the fibers can be biodegradable over time, such that it will be absorbed into a subject if implanted in a subject. Woven fiber substrates, which are biodegradable, can be formed from monomers, such as glycolic acid, lactic acid, propyl fumarate, caprolactone, hyaluronan, hyaluronic acid, and the like. Other fiber substrates can include proteins, polysaccharides, polyhydroxy acids, polyorthoesthers, polyanhydrides, polyphosazenes, or synthetic polymers (particularly biodegradable polymers). In some embodiments, polymers for forming the fiber substrates can include more than one monomer (*e.g.,* combinations of the indicated monomers). Further, the fiber substrate can include hormones, such as growth factors, cytokines, and morphogens (*e.g.,* retinoic acid, arachidonic acid, etc.), desired extracellular matrix molecules (*e.g.,* fibronectin, laminin, collagen, etc.), or other materials (e.g., DNA, viruses, other cell types, etc.) as desired.

Polymers used to form the fibers can include single polymer, co-polymer or a blend of polymers of poly(L-lactic acid), poly(DL-lactic acid), polycaprolactone, poly(glycolic acid) or polyanhydride. Naturally occurring polymers can also be used such as reconstituted or natural collagens or silks. Those of skill in the art will understand that these polymers are just examples of a class of biodegradable polymers that can be used in the presently disclosed subject matter. Further biodegradable polymers include polyanhydrides, polyorthoesters, and poly(amino acids).

Examples of natural polymers that can be used for the fibers include naturally occurring polysaccharides, such as, for example, arabinans, fructans, fucans, galactans, galacturonans, glucans, mannans, xylans (such as, for example, inulin), levan, fucoidan, carrageenan, galatocarolose, pectic acid, pectins, including amylose, pullulan, glycogen, amylopectin, cellulose, dextran, dextrin, dextrose, glucose, polyglucose, polydextrose, pustulan, chitin, agarose, keratin, chondroitin, dermatan, hyaluronic acid, alginic acid, xanthan gum, starch and various other natural homopolymer or heteropolymers, such as those containing one or more of the following aldoses, ketoses, acids or amines: erythrose, threose, ribose, arabinose, xylose, lyxose, allose, altrose, glucose, dextrose, mannose, gulose, idose, galactose, talose, erythrulose, ribulose, xylulose, psicose, fructose, sorbose, tagatose, mannitol, sorbitol, lactose, sucrose, trehalose, maltose, cellobiose, glycine, serine, threonine, cysteine, tyrosine, asparagine, glutamine, aspartic acid, glutamic acid, lysine, arginine, histidine, glucuronic acid, gluconic acid, glucaric acid, galacturonic acid, mannuronic acid, glucosamine, galactosamine, and neuraminic acid, and naturally occurring derivatives thereof. Accordingly, suitable polymers can include, for example, proteins, such as albumin.

Examples of semi-synthetic polymers that can be used to form the fibers include carboxymethylcellulose, hydroxymethylcellulose, hydroxypropylmethylcellulose, methylcellulose, and methoxycellulose. Exemplary synthetic polymers include polyphosphazenes, polyethylenes (such as, for example, polyethylene glycol (including the class of compounds referred to as PLURONICS, commercially available from BASF, Parsippany, N.J., U.S.A.), polyoxyethylene, and polyethylene terephthlate), polypropylenes (such as, for example, polypropylene glycol), polyurethanes, polyvinyl alcohol (PVA), polyvinyl chloride and polyvinylpyrrolidone, polyamides including nylon, polystyrene, polylactic acids, fluorinated hydrocarbon polymers, fluorinated carbon polymers (such as, for example, polytetrafluoroethylene), acrylate, methacrylate, and polymethylmethacrylate, and derivatives thereof.

In some embodiments, the fibers can be assembled into a three dimensional fiber substrate, or textile substrate using a 3-D computer controlled weaving loom, such as a jacquard loom, specifically constructed to produce precise structures from fine diameter fibers. The weaving pattern of the woven substrate, textile, or fabric is defined by the fiber assembly pattern or weaving algorithm designed from the intrinsic pattern of the mapped mechanical properties, material properties, structural molecules, e.g., structural protein fibers of the extracellular matrix of the biologic material.

The weaving pattern and/or weaving algorithm can also use or incorporate spatial and temporal patterns of (in-)elasticity to create dynamic pressures, such as described in WO2015/021503. The textile at least one region of temporally-controlled elasticity may include a step of weaving threads having varying composition and/or elasticity along their length into the substrate.

The textile substrate and/or composite textile formed therefrom can have a region of temporally-controlled elasticity that transitions between a first state and a second state in response to the external stimuli. The first state can be more relaxed than the second state, and the smart material can at least partially revert from the second state to the first state over an extended time period resulting from the temporally-controlled elasticity of the textile substrate. The internal energy of the smart material in the first state can be less than internal energy of the substrate in the second state. Different regions of the smart material can possess different temporally-controlled elasticity.

In some embodiments, the textile substrate and/or composite textile formed therefrom can move from the second state to the first state via any one of elongation or shortening of the smart material, or relaxation or stiffening of the smart material. The textile substrate and/or composite textile formed therefrom can possess spatially-controlled elasticity, whereby different regions of the textile substrate have different elasticity or stiffness.

In some embodiments, the textile substrate can be woven using at least two threads/fibers, wherein each thread has a different elasticity.

In other embodiments, the textile substrate can include at least one thread possessing elasticity that varies along the length of the thread. The textile substrate can include at least one thread possessing elasticity that varies within the cross-section of the thread.

In other embodiments, the textile substrate can be woven using threads arranged in different directions such that the threads move frictionally relative to one another causing the transition from the first state to the second state to occur over an extended time period.

A computer controlled weaving machine can produce true 3-D shapes by placing fibers axially (x-warp direction), transversely (y-weft, or filling direction), and vertically (z-thickness direction). Multiple layers of warp yarns are separated from each other at distances that allow the insertion of the weft layers between them. Two layers of Z-yarns, which are normally arranged in the warp direction, are moved (after the weft insertion) up and down, in directions opposite to the other. This action is followed by the "beat-up", or packing of the weft into the scaffold, and locks the two planar fibers (the warp and weft) together into a uniform configuration. Change of yarn densities can be achieved for warp by altering the reed density and warp arrangement and for weft by varying the computer program controlling the take-up speed of a stepper motor.

An advantage of the presently disclosed weaving technique is that each fiber can be selected individually and woven into a textile substrate. Using this method of assembly, customized structures can be easily created by selectively placing different constituent fibers *(e.g.,* fibers of various material composition, size, and/or coating/treatment) throughout the textile substrate. In this manner, physical and mechanical properties of the textile substrate can be controlled (*i.e.,* pore sizes can be selected, directional properties can be varied, and discreet layers can be formed). Using this technique, the inhomogeneity and anisotropy of various tissues can be reproduced by constructing a textile substrate that mimics the normal stratified structural network using a single, integral textile substrate.

In some embodiments, the fibers can be provided as threads that are oriented in space relative to each other during the assembly step. The assembly step includes can including orienting threads having different elasticity along their length according to a predetermined algorithm.

In other embodiments, yarns of the fibers after assembly can be set via any of a number of art-recognized techniques, including but not limited to ultrasonication, a resin, infrared irradiation, heat, or any combination thereof. Setting of the yarn systems within the scaffold in this manner provides cuttability and suturability. Sterilization can be performed by routine methods including, but not limited to autoclaving, radiation treatment, hydrogen peroxide treatment, ethylene oxide treatment, and the like.

Representative methods for making three-dimensional textile substrates are also disclosed in U.S. Pat. Nos. 5,465,760 and 5,085,252. It is also referred to the following patent publications: PCT International Patent Application Publication WO 01/38662 (published May 31, 2001); PCT International Patent Application Publication WO 02/07961 (published Jan. 31, 2002); U.S. Patent Application Publication 2003/0003135 (published Jan. 2, 2003), and PCT International Patent Application Serial No. PCT/US06/14437, filed Apr. 18, 2006.

Following or during formation of the textile substrate, a material can deposited via an additive manufacturing technique onto and/or between the fibers of the textile substrate based on an additive manufacturing pattern to form a composite textile that includes a gradient in least one of mechanical property, material property, or structural property and/or exhibits a change in at least one mechanical property, material property, or structure in response to at least one external stimulus.

The material deposited via the additive manufacturing technique can include any known inorganic or organic material that can be deposited using additive manufacturing techniques. Such materials can include, for example, plastics or polymers, epoxies, elastomers, reactive polymer systems (e.g., polyurethane, polyurea), preceramic polymer resins, ceramics, metals, bio-materials, gels, and/or inks.

In some embodiments, the plastics or polymers can include aliphatic, polycarbonate based thermoplastic polyurethanes, thermoplastic elastomers, polytetramethylene glycol based polyurethane elastomers, polyethylene naphthalate and isomers thereof, such as 2,6-, 1,4-, 1,5-, 2,7-, and 2,3-polyethylene naphthalate; polyalkylene terephthalates such as polyethylene terephthalate, polybutylene terephthalate, and poly-1,4-cyclohexanedimethylene terephthalate; aromatic polyesters, polyimides, such as polyacrylic imides; polyetherimides; styrenic polymers, such as atactic, isotactic and syndiotactic polystyrene, α-methyl-polystyrene, para-methyl-polystyrene; polycarbonates such as bisphenol-A-polycarbonate (PC); poly(meth)acrylates such as glassy poly(methyl methacrylate), poly(methyl methacrylate), poly(isobutyl methacrylate), poly(propyl methacrylate), poly(ethyl methacrylate), poly(butyl acrylate) and poly(methyl acrylate) (the term "(meth)acrylate" is used herein to denote acrylate or methacrylate); cellulose derivatives, such as ethyl cellulose, cellulose acetate, cellulose propionate, cellulose acetate butyrate, and cellulose nitrate; polyalkylene polymers, such as polyethylene, polypropylene, polybutylene, polyisobutylene, and poly(4-methyl)pentene; fluorinated polymers, such as perfluoroalkoxy resins, polytetrafluoroethylene, fluorinated ethylene-propylene copolymers, polyvinylidene fluoride, and polychlorotrifluoroethylene and copolymers thereof; chlorinated polymers, such as polydichlorostyrene, polyvinylidene chloride and polyvinylchloride; polysulfones; polyethersulfones; polyacrylonitrile; polyamides; polyvinyl acetate; aromatic polyamides (*e.g.,* amorphous nylons, such as Dupont Sellar or EMS G21), and polyether-amides as well as natural polymer macromers, such as poly(saccharide), poly(HEMA), collagen, fibrin, gelatin, glycosaminoglycans (GAG), poly (hyaluronic acid), poly(sodium alginate), alginate, hyaluronan, agarose, copolymers thereof, and blends thereof.

Examples of inorganic materials include metal, semiconductor, and or non-metal materials, such as bismuth ferrite (BiFeO₃), cadmium sulfide (CdS), cadmium telluride (CdTe), fullerenes (C60), graphite, graphene oxide, carbon nanoparticles, zinc oxide (ZnO) titanium dioxide (TiO₂) particles, metal particles, metal coated particles, inorganic oxides, metal oxides, and combinations thereof

The material can be deposited onto and/or between fibers of the textile substrate using any additive manufacturing technique based on the additive manufacturing pattern. The additive manufacturing technique can include, for example, one or more of a fused deposition modeling (FDM) technique, a fused filament fabrication (FFF) technique, a big area additive manufacturing (BAAM) technique, a robocasting technique, an electrospinning technique, a paste extrusion technique, and/or a direct ink writing (DIW) technique.

In one example, the material can be deposited onto and/or between fibers of the textile substrate using 3D printing. 3D printing has conventionally been used to create static objects and other stable structures, such as prototypes, products, and molds. Three-dimensional printers can convert a 3D image, which is typically created with computer-aided design (CAD) software, into a 3D object through the layer-wise addition of material.

One example of such a 3D printing technology includes multi-material three-dimensional (3D) printing technologies, which allow for deposition of material patterns with heterogeneous composition. For example, 3D printed structures can be composed of two or more materials having particular physical and chemical properties. Examples of 3D printers that can be used for the 3D printing of multi-material objects are described in U.S. Pat. Nos. 6,569,373; 7,225,045; 7,300,619; and 7,500,846; and U.S. Patent Application Publication Nos. 2013/0073068 and 2013/0040091. Printing of materials having a variety of properties, including rigid and soft plastics and transparent materials, and provide high-resolution control over material deposition. One of skill in the art will understand that it may be necessary to cure (*e.g.,* polymerize) the 3D printed material.

The additive manufacturing pattern used for printing of the material can be designed by reference to a predetermined 3D geometric shape. In some embodiments, the additive manufacturing pattern can be based on the mapped three dimensional spatial distribution of at least one mechanical property, material property, or structure of a natural or biological material of interest. In some embodiments, the additive manufacturing pattern can include a printing algorithm or printing motif based on the intrinsic pattern of the mapped three dimensional spatial distribution of at least one mechanical property, material property, or structural property as well as other structural features. In some instances, the intrinsic pattern can be used to design or generate a custom-configured printing algorithm.

As indicated above, the deposited material defines a matrix that includes a plurality of pores with a hierarchal porosity and/or porosity pattern in the composite textile. Furthermore, the additive manufacturing pattern for the deposited material is based on a three dimensional spatial distribution of pores in a biological material of interest.

In other embodiments, a fluid *(e.g.,* liquid) can be provided within the pores of the composite textile. In some embodiments, the movement of the fluid in the pores can be used dissipate energy in response to force or impact on and/or of the composite textile. For example, body armor can be formed from a composite textile that includes a woven fiber substrate on which is deposited a material matrix that includes a hierarchal porosity and/or porosity gradient and/or porosity pattern. The porosity of matrix and composite textile can be such that fluid provided in the pores can dissipate impact energy or force from projectile striking the body armor.

In other embodiments, the pores can have a hierarchy and/or gradient such that composite textile includes a first region that exudes fluid in response to a compressive or tensile load and a second region that imbibes fluid in response to the load similar to the flow directing material disclosed in U.S. Patent Application No. 12/106,748 to Knothe Tate et al. The flow directing material has a porous structure and is capable of being compressed when a load is applied to the outer surfaces of the material. By way of example, the matrix defined by the deposited material can be a porous compliant polymeric material that includes a first region and the second region that extend from an outer surface of the composite textile. In response to compressive or tensile load to the composite textile, the first region can exude fluid from the outer surface toward the direction of the load, and the second region can imbibe fluid from the outer surface away from the direction of the load.

In some embodiments, the exuding region can have a first porosity, and the imbibing region can have a second porosity. The porosities (or porosity ratio *(e.g.,* void volume of the respective region in mm³/total volume of the respective region in mm³)) of the exuding region and the imbibing region can be about 0.3 and about 0.7, respectively. The porosities of the exuding region and the imbibing region can also be at least about 5% different so that the direction of fluid flow in and/or through the exuding region will be different than (*e.g.,* contrary, opposite, and/or substantially normal to) the direction of fluid flow in and/or through the imbibing region. That is, the difference of porosities of the exuding region and the imbibing region can determine, at least in part, the direction of fluid flow in and/or through the exuding region and the imbibing region.

The exuding region and the imbibing region can also have, respectively, a first permeability and a second permeability. The permeabilities of the exuding region and the imbibing region can be about 10⁻¹³ m² to about 10⁵ m². The permeability can control the magnitude of fluid flow in the composite textile, when the composite textile is under compression, and can potentially control the timing of transport of fluid depending on the specific application of the composite textile. In one aspect, the exuding region can have substantially the same permeability as the imbibing regions. In another aspect, the exuding region and the imbibing regions can have different permeabilities.

In other embodiments, the composite textile can include a plurality of first regions laterally spaced from one another in the composite textile and separated by the second region. At least some of the first regions can have a different porosity, volume, volumetric permeability, and/or surface permeability than the porosity, volume, volumetric permeability, and/or surface permeability of other first regions.

In some embodiments, the composite textile so formed can be used to generate engineered tissue implant or mechanically functional textiles, which can be used to treat and/or repair tissue defects, such as bone defects or soft tissue defects. The composite textile can be used in its native form in combination with other materials, as an acellular (non-viable) matrix, or combined with at least one cell and/or at least one bioactive agents (*e.g.,* growth factors) for use in repair, regeneration, and/or replacement of diseased or traumatized tissue and/or tissue engineering applications. An advantage of the presently disclosed subject matter is the ability to produce biomaterial scaffolds and composite matrices that have precisely defined mechanical properties that can be inhomogeneous (vary with site), anisotropic (vary with direction), nonlinear (vary with strain), and/or viscoelastic (vary with time or rate of loading) and that mimic native or natural tissue to be treated and/or repaired.

In other embodiments, the at least one bioactive agent provided in the composite textile can include polynucleotides and/or polypeptides encoding or comprising, for example, transcription factors, differentiation factors, growth factors, and combinations thereof. The at least one bioactive agent can also include any agent capable of promoting tissue formation (*e.g.,* bone and/or cartilage), destruction, and/or targeting a specific disease state (*e.g.,* cancer). Examples of bioactive agents include chemotactic agents, various proteins (*e.g.,* short term peptides, bone morphogenic proteins, collagen, glycoproteins, and lipoprotein), cell attachment mediators, biologically active ligands, integrin binding sequence, various growth and/or differentiation agents and fragments thereof (*e.g.,* EGF), HGF, VEGF, fibroblast growth factors (*e.g.,* bFGF), PDGF, insulin-like growth factor (*e.g.,* IGF-I, IGF-II) and transforming growth factors (*e.g.,* TGF-β I-III), parathyroid hormone, parathyroid hormone related peptide, bone morphogenic proteins (*e.g*., BMP-2, BMP-4, BMP-6, BMP-7, BMP-12, BMP-13, BMP-14), sonic hedgehog, growth differentiation factors *(e.g.,* GDF5, GDF6, GDF8), recombinant human growth factors *(e.g.,* MP-52 and the MP-52 variant rhGDF-5), cartilage-derived morphogenic proteins (CDMP-1, CDMP-2, CDMP-3), small molecules that affect the upregulation of specific growth factors, tenascin-C, hyaluronic acid, chondroitin sulfate, fibronectin, decorin, thromboelastin, thrombin-derived peptides, heparin-binding domains, heparin, heparan sulfate, polynucleotides, DNA fragments, DNA plasmids, MMPs, TIMPs, interfering RNA molecules, such as siRNAs, DNA encoding for an shRNA of interest, oligonucleotides, proteoglycans, glycoproteins, and glycosaminoglycans.

The at least one cell provided in the composite textile can include any progenitor cell, such as a totipotent stem cell, a pluripotent stem cell, or a multipotent stem cell, as well as any of their lineage descendant cells, including more differentiated cells (described above). The cells can include autologous cells; however, it will be appreciated that xenogeneic, allogeneic, or syngeneic cells may also be used. Where the cells are not autologous, it may be desirable to administer immunosuppressive agents in order to minimize immunorejection. The cells employed may be primary cells, expanded cells, or cell lines, and may be dividing or non-dividing cells. Cells may be expanded *ex vivo* prior to introduction into the woven fiber substrate, textile, and/or fabric. For example, autologous cells can be expanded in this manner if a sufficient number of viable cells cannot be harvested from the host. Alternatively or additionally, the cells may be pieces of tissue, including tissue that has some internal structure. The cells may be primary tissue explants and preparations thereof, cell lines (including transformed cells), or host cells.

In some embodiments, the composite textile can be mixed or embedded with cells before or after implantation into the body. The composite textile can function to provide a template for the integrated growth and differentiation of the desired tissue.

In some embodiments, the cells are introduced into pores of the composite textile or textile substrate, such that they permeate into the interstitial spaces therein. For example, the composite textile or textile substrate can be soaked in a solution or suspension containing the cells, or they can be infused or injected into the matrix of the textile substrate. As would be readily apparent to one of ordinary skill in the art, the composition can include mature cells of a desired phenotype or precursors thereof, particularly to potentate the induction of the stem cells to differential appropriately within the composite (e.g., as an effect of co-culturing such cells within the composite).

In some embodiments, the composite textile can be coated on one or more surfaces, before or after consolidation with cells, with a material to improve the mechanical, tribological, or biological properties of the textile composite. Such a coating material can be resorbable or non-resorbable and can be applied by dip-coating, spray-coating, electrospinning, plasma spray coating, and/or other coating techniques. The material can be a single or multiple layers or films. The material can also comprise randomly aligned or ordered arrays of fibers. In some embodiments, the coating can comprise electrospun nanofibers. The coating material can be selected from the group including, but not limited to polypropylene, polyester, polytetrafluoroethylene (PTFE), expanded PTFE (ePTFE), polyethylene, polyurethane, polyamide, nylon, polyetheretherketone (PEEK), polysulfone, a cellulosic, fiberglass, an acrylic, tantalum, polyvinyl alcohol, carbon, ceramic, a metal, polyglycolic acid (PGA), polylactic acid (PLA), polyglycolide-lactide, polycaprolactone, polyethylene glycol) (PEG), polydioxanone, polyoxalate, a polyanhydride, a poly(phosphoester), catgut suture, collagen, silk, chitin, chitosan, hydroxyapatite, bioabsorbable calcium phosphate, hyaluronic acid, elastin, lubricin, and combinations thereof.

In some embodiments, a smooth surface coat on the composite textile is thus provided if needed. In some embodiments, the surface coat can increase durability and/or reduce friction of and/or at the surface.

In some embodiments, the composite textile can be employed in any suitable manner to facilitate the growth and generation of desired tissue types or structures. For example, the composite textile can be constructed using three-dimensional or stereotactic modeling techniques. Thus, for example, a layer or domain within the composite textile can be populated by cells primed for one type of cellular differentiation, and another layer or domain within the composite textile can be populated with cells primed for a different type of cellular differentiation. As disclosed herein and as would be readily apparent to one of skill in the art, to direct the growth and differentiation of the desired structure, in some embodiments, the composite textile can be cultured *ex vivo* in a bioreactor or incubator, as appropriate. In some embodiments, the structure is implanted within the subject directly at the site in which it is desired to grow the tissue or structure. In further embodiments, the composite textile can be grafted on a host *(e.g.,* an animal such as a pig, baboon, etc.), where it can be grown and matured until ready for use, wherein the mature structure is excised from the host and implanted into the subject.

It will be appreciated that the composite textile can be used in a variety of engineered smart materials bespoke external (wearable) and internal (implants, medical devices) wound dressings that deliver drugs and take up wound exudate.

In some embodiments, a wound dressing can be formed from a composite textile that includes a textile substrate and a porous matrix. The textile substrate can have a region of temporally-controlled elasticity that transitions between a first state and a second state in response to the external stimuli. The first state can be more relaxed than the second state, and the smart material can at least partially revert from the second state to the first state over an extended time period resulting from the temporally-controlled elasticity of the textile substrate. The internal energy of the smart material in the first state can be less than internal energy of the substrate in the second state. Different regions of the smart material can possess different temporally-controlled elasticity.

The porous matrix of the composite textile can both imbibe excess fluid or exudate from a wound and exudes therapeutic agents to the wound when the dressing is under compression. In some embodiments, as illustrated in Fig. 8, the substrate includes a plurality of laterally spaced exuding regions in the form of cylindrical dots that under compression exude a therapeutic fluid. The material surrounding the dots can imbibe excess fluid or exudate when the dressing is compressed against the wound. The exuding regions have a first porosity and a first permeability. The imbibing surrounding region has a second porosity and a second permeability.

The exuding regions of the dressing can include depots (not shown) that contain the therapeutic fluid in the exuding regions. The therapeutic fluid can flow from the exuding regions through a delivery surface when the dressing is under compression. The therapeutic fluid can include at least one pharmaceutical agent, anti-inflammatory agent, antibiotic, antifungal agent, antipathogenic agent, antiseptic agent, hemostatic agents, local analgesics, immunosuppressive agents, growth factor, peptide, or gene therapy agent. The second imbibing region can imbibe excess fluid or exudate from the wound or skin of the subject when the delivery surface of the dressing is applied against the wound or skin of the subject and compressed.

The exuding regions of the dressing can comprise a first porous polymeric material having a first porosity. The surrounding imbibing region can comprise a second porous polymeric material having a second porosity different than the first porosity. The first porous polymeric material can have a first flexible polymeric foam structure of interconnected open cells. The second porous polymeric material can have a second flexible polymeric foam structure of interconnected open cells.

The dressing can also include a slip layer attached to the outer surface of the substrate. The slip layer can minimize friction of the dressing with the outer environment when the dressing is applied to a wound of the subject.

The composite dressing can deliver therapeutic substances through the delivery dots and imbibe fluid through the surrounding material surrounding the dots. The composite dressing can also be designed and/or deliver substances through the larger volume material surrounding the dots and imbibe fluid through the smaller volume of the dots.

It will be appreciated that the composite textile can be used in the formation of a variety of smart materials where it is desired to control or modulate mechanical properties of the material and/or control fluid flow of the material. Such smart materials can include body armor, tissue constructs, and wound dressings as described herein as well as other materials, such as flooring material, where it is desirable to provide strength in tension and bending with smart poroelastic properties, found in flow directing materials. Additionally, smart materials including the composite textiles can be used to form wearables, such as clothing, garments, or dressings, that can dynamically apply pressure in various points of the body to increase or decrease blood flow, imbibe or exude moisture, based on external stimuli.

### Example

This example describes a microscopy aided design and manufacture (MADAME) technology platform that is used to engineer and manufacture materials, products, and devices that emulate the smart mechanical and transport properties of nature's own (Fig. 1). Nature abounds with advanced, stimuli responsive materials that if emulated, provide new solutions to currently untenable design problems. Such problems include the discrepancy between the human life span and the design life of the human hip and its contemporary implant replacement. Human joints offer complex geometrical solutions to increase range of motion and stability during daily activities, *e.g.,* ball and socket for the hip or complex composite bone and composite bone and ligamentous structure of the plane synovial acromioclaviular joint. Yet, novel design solutions may emulate emergent properties of natural joints and springs. For example, the eucalyptus tree exhibits a gradient in mechanical properties, enabling it to bend like a blade of grass under gale force winds while transporting nutrients upwards of 100 meters from the roots to the tip. At a different length scale, the grasshopper knee also exhibits gradients enabling "jointedness" and an intrinsic leaf spring. While 3D printing offers advantages with regard to rapid manufacturing materials and parts with mechanical gradients, it shows distinct disadvantages in particular for parts exposed to bending and tension. Recent advances in 4D printing incorporate actuator and sensor functions intrinsic to i.a. piezoelectric properties of 3D printed pieces, engineering of residual stresses into parts that can transform their geometry reversibly via folding. One such disruptive 4D printing modality harnesses natural movements, *e.g.,* of the wearer or attributable to nature's cycles (tidal, weather, seasons, etc.), to design novel wearables and smart systems. MADAME uses computer-aided additive manufacturing incorporating three dimensional (4D) printing and computer-controlled weaving to create composite design motifs that emulate tissue patterns of woven protein fibers, gradients in different caliber porosities, and mechanical and molecular properties intrinsic to tissues. In so doing, MADAME enables a new genre of smart materials, products and replacement body parts that exhibit advantageous properties in bending and tension as well as in compression and materials that harness forces linked to physiological activity to activate material properties.

### Recursive Logic and Weaving of Textiles with Biophysical and Spatiotemporal Patterns

MADAME describes the novel process of mapping spatial and temporal properties intrinsic to nature's smart materials, using imaging, and advanced computational methods (Figs. 1, 2). The patterns intrinsic to such materials are then recreated using recursive logic. Remarkably, the loom was the earliest computer-prior to the first punch card driven computers, the Jacquard loom wove patterns using loops of paper with holes to guide when hooks fell through the paper loop (hook down) or stayed above the loop (hook up), thereby encoding binary patterns of *e.g.,* tapestry weaves. Recursive logic provides a basis for computer coding algorithms and computer-controlled Jacquard looms enable creation of physical embodiments (textiles) of mechanical and other biophysical and spatiotemporal patterns intrinsically encoded in natural materials.

The MADAME technology was developed to emulate the intrinsic weaves of natural tissues, from tree bark to grasshopper joints to human skin and bones. As an example, the patterns of structural proteins including elastin and collagen which imbue tissues with their respective elastic and toughness properties can be recursively mapped out and then imported into computer aided design files to weave textiles with scaled up mechanical property patterns mimicking those of the natural tissue (Fig. 3). In this way, the Jacquard loom technology provides a platform to create patterns of a variety of biophysical properties instead of its traditional use for the creation of color patterns in fabric and/or tapestries. Modern computer-controlled looms provide a rapid manufacturing method enabling control over 5,000 individual fibers, which themselves have different physical properties such as elasticity, respectively, stiffness. Composite materials are thus created in combination with 3D printing.

### Mapping of Hierarchical Porosities in Natural Tissues

An aspect of MADAME is the quantification and visualization of several orders of magnitude different length scale features within the same natural sample, which is often studied in the form of a histological section. The process from which patterns are derived from biological samples can involve recursive logic, as previously described, or clever image analysis approaches to identify and separate out (segment) different sized features, after which gradients can be described spatially, *e.g.,* as heat maps, to better visualize their distribution in space and in relationship to each other.

In addition to the importance of mechanical property gradients in natural materials, porosity gradients provide transport pathways while also modulating mechanical properties of natural materials. For example, bone exhibits at least three levels of hierarchical porosity and gradients thereof which are characteristic to the tissue and which imbue the tissue with remarkable smart properties, such as counterintuitive flow properties (exuding fluid under compression and imbibing fluid under tension), and flow directing transport areas of the tissue that are poorly vascularized, as well as providing direct conduits (resorption cavities created by osteoclasts) for osteoblasts to penetrate and lay down new bone in an oriented fashion, achieving anisotropic structural stability similar to reinforced concrete.

Automated segmentation and mapping of different calibers of porosity within the sample is a non-trivial problem. In the following case study, we address the problem in detail for clarity and to allow for reduction to practice using different imaging modalities. To analyze porosity of whole bone cross sections andmultiple length scales, enabling spatialmapping and analysis of vascular porosity and pericellular porosity, a computer algorithm was developed in MATLAB (MathWorks, Inc., Natick, MA, United States). First, the vascular porosity of bone was mapped. High resolution confocal microscopy collages were acquired for the entire cross section of a histological sample containing a rat ulna and radius which had been injected intravitally with a 300 Da fluorescent tracer (Fig. 4). Vessels were identified automatically using the MATLAB algorithm and a mask of bone devoid of vessels was created to segment bone and calculate internal porosity. In this particular sample, the vascular porosity made up 2.46% of the cross sectional area of bone (Fig. 4).

To calculate the cell-length scale lacunar porosity (the lacunae are the voids in which the cells reside), transmitted light images were used similar to the way that the confocal images were used to calculate vascular porosity in the previous example. A mask was created, first without porosity, and then the lacunar porosity was calculated in 100 micron thick samples. The different caliber pores were identified as vessels and lacunae, while also accounting for the volume (Figs. 4E,F). The lacunar porosity was calculated by generating a mask without porosity, and calculating the number of lacunae (Figs. 5A,B), resulting in a lacunar porosity of 1.1% for the example. This process was then carried out for specific areas around the cross section to determine the site specific lacunar porosity (Figs. 5C1-5).

Then the site specific distribution of the vascular and lacunar porosities that make up the transport pathways were mapped using collages of high resolution confocal images (Figs. 6A-C), which are depicted as "heatmaps" (Fig. 7). The logic underpinning the "heatmaps" forms the basis of a MatLab algorithm. In short, the measured porosity values are displayed in the form of color contour plots. These plots resemble the false color images obtained from imaging. MATLAB stores most images as two-dimensional arrays (i.e., matrices), in which each element of the matrix corresponds to a single pixel in the displayed image. A matrix with exactly the same dimension as the input image comprises all zero values. Next a randomly chosen region in the image is analyzed and two outputs are calculated including number of lacunae per area and vascular pores per area. These two parameters are then linked to the region in a way that the values are assigned to every matrix element representing the randomly selected area. Repeating this procedure several times causes regions to overlap (Fig. 6D). Overlapping regions are averaged (Fig. 7A), which leads to a good representation of the output-data over the cross-section if enough iterations are performed. In this way, a heat map of density of pores of two different calibers is created for the entire cross section, with warm colors depicting areas of high density and cool colors depicting areas of low density of *e.g.,* lacunar and vascular porosity (Figs. 7C, D).

This algorithm can be used to co-register images and their collages from imaging modalities as diverse as confocal laser imaging (yielding *e.g.,* porosity gradients), second harmonic imaging (yielding *e.g.,* collagen and elastin fiber gradients), atomic force and electron microscopy, multibeam scanning electron microscopy, computed tomography, magnetic resonance imaging, etc. These data sets, when encoded in computer aided design and computer aided manufacture file formats, serve as inputs for combined weaving of fiber patterns and multidimensional advanced manufacture (*e.g.,* 3D printing or laser sintering) of porous structures. This enables creation of composite materials with strength in tension and bending and with smart, poroelastic properties, such as flow directing materials. Hence, MADAME can be used to create novel materials and parts with gradients in poroelastic properties emulating those found in smart, natural materials.

### Additive Manufacturing of Scaled Up Natural Properties, Including Pore Gradients

Encoded in computer aided design and computer aided manufacture file formats, *e.g.,* stereolithography (stl) or 3D Manufacturing Format (3MF) files, spatial plots of features provide inputs for additive manufacturing of materials, products, and parts that exhibit gradients and/or distributions in properties of natural materials. Additive manufacturing can take place via either computer-controlled weaving and/or additive manufacturing processes including, for example, stereolithography, powder sintering, 3D printing, etc. and/or electrospinning, weaving, and knitting.

The order and/or combined processes of weaving, knitting and spinning with 3D printing can be tuned to achieve the desired final properties of the materials, products and parts. For example, a weave can be placed within a stereolithography bath, enabling polymerization of polymeric matrix in gradients defined by scaled microscopy data around the weave. Similarly, with laser sintering, apatite and other mineral or metal based powders can be sintered around the weave. Integrated weaving and 3D systems will enable the weaving of textiles within the monomer baths using jets instead of hook-based weaving looms that are completely integrated with 3D printing modalities.

Thus, we have described a pipeline or machine-based workflow (Fig. 2) to design and manufacture smart dressings, drug delivery patches, and replacement body parts using MADAME. MADAME shows great promise for the realization of new classes of materials, products and devices that will benefit patients, allowing for incorporation of unprecedented spatial and temporal patterns. One example is a new class of "designer" wound dressings cum delivery devices that are tuned to the spatial and temporal wound healing and drug release kinetics of individual patients, that harness the patient's movements to facilitate delivery, and that signal the wearer or the carer when the active ingredients are spent (Figure 8). This application can be further expanded for development of new classes of wearable materials and devices as well as internal applications, such as implants and medical devices.

The pipeline has been tested on scaled up, three dimensional confocal microscopy datasets of the pericellular space in cortical bone (Figure 9). In this case, volumetric microscopy data was inverted to represent the fluorescent-dye infused cellular features as voids, and approximated in stl file format. The stl files contain no scale information, i.e., can be scaled up or down and used as inputs to create physical renderings at any desired scale and using any compatible rapid manufacturing modality. The physical renderings thus created, *e.g.,* via 3D printing, enable unprecedented measurements using similitude theory, where measures at actual length scale are scaled up and down from the physical rendering. Similitude is a powerful, classical tool in mechanical engineering, applied by Da Vinci through to the modern day. In the current example of the pericellular fluid space in cortical bone, for the first time pericellular tissue permeability could be measured on scaled up physical renderings of actual tissues. Pericellular permeability measures are of particular relevance for predicting of pharmaceutical delivery kinetics at local and global length scales.

Similarly, the pipeline was tested and validated in scaled up patterns of structural proteins mapped in ovine periosteum, an elastic and soft tissue sheath covering all bone surfaces and providing a niche for stem cells. For the first time, using MADAME it was possible to create textiles that emulate the smart mechanical properties of the periosteum. The value proposition of MADAME is to scale up gradients in, for example, mechanical properties, porosities, and protein patterns to rapid prototype new materials that emulate patterns in natural materials. This provides an unprecedented means by which smart properties of natural tissues and systems can be mapped precisely using high resolution microscopy and used as a basis for manufacturing of scaled up materials that emulate nature systems.

The pipeline can be further tailored to best harness the wearer's natural movements and thereby to *e.g.,* augment transport to and from the wound surface via material design that directs convective flow by harnessing displacements at the interface with the skin (Figs. 8, 10). Thus, MADAME integrates inputs encoding material properties in context of the physiological mechanical environment in which the thus designed and manufactured products will be used, which provides independent and synergistic optimization of materials design and manufacture.

The inherent advantages and disadvantages of the MADAME technology align with those of current 3D- and 4D-printing technology platforms. The major advantage of MADAME over current 3D- and 4D-printing modalities is that provides a means to manufacture novel composites with biophysical and spatiotemporal gradients and associated sensor and actuator functions that harness natural movements or transformations. The major disadvantages of MADAME include the need for high resolution imaging that crosses length scales, as well as cutting edge testing and validation, both of which requires operators with multidisciplinary, technical, and soft skillsets.

## Claims

1. A smart material comprising:
a composite textile that includes a textile substrate formed from a plurality of fibers assembled in a fiber assembly pattern; and
a material deposited via an additive manufacturing technique onto and/or between the fibers of the textile substrate based on an additive manufacturing pattern, wherein the composite textile includes a gradient in least one of mechanical property, material property, or structural property and/or exhibits a change in at least one mechanical property, material property, or structure in response to at least one external stimulus,
wherein the additive manufacturing pattern is based on a three dimensional spatial distribution of pores in a biological material of interest and the deposited material defines a matrix that includes a plurality of pores with a hierarchical porosity and/or porosity pattern in the composite textile.

2. The smart material of claim 1, wherein the fiber assembly pattern is based on a three dimensional spatial distribution of at least one structural molecule of the biological material of interest; and the fibers are assembled based on the fiber assembly pattern to form the textile substrate.

3. The smart material of claim 2, wherein the structural molecule comprises at least one structural protein fiber of the extracellular matrix.

4. The smart material of claim 3, wherein the at least one structural protein fiber comprises collagen fibers and elastin fibers of the extracellular matrix of the biological material of interest, wherein the assembled fibers are preferably woven using a weaving algorithm based on the three dimensional spatial distribution of the at least one structural protein fiber to define the weave pattern and fiber orientation.

5. The smart material of claim 1, wherein the biological material of interest comprises tissue of a plant or animal.

6. The smart material of claim 1, wherein additive manufacturing technique comprises one or more of a fused deposition modeling (FDM) technique, a fused filament fabrication (FFF) technique, a big area additive manufacturing (BAAM) technique, a robocasting technique, a paste extrusion technique, an electrospinning technique, and/or a direct ink writing (DIW) technique.

7. A method of forming a smart composite textile, the method comprising:
mapping a three dimensional spatial distribution of pores of a biological material of interest;
designing an additive manufacturing pattern based on the three dimensional spatial distribution of pores of the biological material of interest;
assembling a plurality of fibers based on a fiber assembly pattern into a textile substrate;
depositing a material via an additive manufacturing technique onto and/or between fibers of the textile substrate based on the additive manufacturing pattern to provide a composite textile that includes a gradient in least one of mechanical property, material property, or structural property and/or that exhibits a change in at least one mechanical property, material property, or structure in response to at least one external stimulus;
wherein the additive manufacturing pattern is based on a three dimensional spatial distribution of pores in a biological material of interest and the deposited material defines a matrix that includes a plurality of pores with a hierarchical porosity and/or porosity pattern in the composite textile.

8. The method of claim 7, further comprising:
mapping a three dimensional spatial distribution of the structure of the biological material of interest;
designing the fiber assembly pattern based on the three dimensional spatial distribution at least one structural molecule of the biological material of interest; and
assembling fibers based on the fiber assembly pattern to form the textile substrate.

9. The method of claim 8, wherein the structural molecule comprises at least one structural protein fiber of the extracellular matrix.

10. The method of claim 9, wherein the at least one structural protein fiber comprises collagen fibers and elastin fibers of the extracellular matrix of the biological material of interest.

11. The method of claim 10, wherein the fiber assembly pattern comprises a weaving algorithm based on the three dimensional spatial distribution of the at least one structural protein fiber, wherein the assembled fibers are preferably woven using the weaving algorithm to define the weave pattern and fiber orientation.

12. The method of claim 7, wherein additive manufacturing technique comprises one or more of a fused deposition modeling (FDM) technique, a fused filament fabrication (FFF) technique, a big area additive manufacturing (BAAM) technique, a robocasting technique, a paste extrusion technique, and/or a direct ink writing (DIW) technique.

13. The method of claim 7, wherein the additive manufacturing pattern includes a printing algorithm based on the three dimensional spatial distribution of pores.

## Patentansprüche

1. Ein intelligentes Material, umfassend:
ein Verbundtextil, das ein textiles Substrat enthält, das aus einer Vielzahl von Fasern gebildet ist, die in einem Faseranordnungsmuster angeordnet sind; und
ein Material, das mittels eines additiven Fertigungsverfahrens auf und/oder zwischen den Fasern des textilen Substrats auf der Grundlage eines additiven Fertigungsmusters aufgebracht wurde, wobei das Verbundtextil einen Gradienten in mindestens einer der folgenden Eigenschaften aufweist: mechanische Eigenschaft, Materialeigenschaft oder strukturelle Eigenschaft, und/oder als Reaktion auf mindestens einen externen Reiz eine Veränderung in mindestens einer mechanischen Eigenschaft, Materialeigenschaft oder Struktur zeigt,
wobei das additive Fertigungsmuster auf einer dreidimensionalen räumlichen Verteilung von Poren in einem biologischen Material von Interesse basiert und das aufgebrachte Material eine Matrix definiert, die eine Vielzahl von Poren mit einer hierarchischen Porosität und/oder einem Porositätsmuster in dem Verbundtextil umfasst.

2. Das intelligente Material nach Anspruch 1, wobei das Faseranordnungsmuster auf einer dreidimensionalen räumlichen Verteilung mindestens eines Strukturmoleküls des betreffenden biologischen Materials basiert; und die Fasern auf der Grundlage des Faseranordnungsmusters angeordnet werden, um das Textilsubstrat zu bilden.

3. Das intelligente Material nach Anspruch 2, wobei das Strukturmolekül mindestens eine Strukturproteinfaser der extrazellulären Matrix umfasst.

4. Das intelligente Material nach Anspruch 3, wobei die mindestens eine Strukturproteinfaser Kollagenfasern und Elastinfasern der extrazellulären Matrix des biologischen Materials von Interesse umfasst, wobei die angeordneten Fasern vorzugsweise unter Verwendung eines Webalgorithmus gewebt werden, der auf der dreidimensionalen räumlichen Verteilung der mindestens einen Strukturproteinfaser basiert, um das Webmuster und die Faserorientierung zu definieren.

5. Das intelligente Material nach Anspruch 1, wobei das biologische Material von Interesse Gewebe einer Pflanze oder eines Tieres umfasst.

6. Das intelligente Material nach Anspruch 1, wobei das additive Fertigungsverfahren eine oder mehrere der folgenden Techniken umfasst: ein Fused-Deposition-Modeling-Verfahren (FDM), ein Fused-Filament-Fabrication-Verfahren (FFF), ein Big-Area-Additive-Manufacturing-Verfahren (BAAM), ein Robocasting-Verfahren, ein Pastenextrusionsverfahren, ein Elektrospinnverfahren und/oder ein Direct-Ink-Writing-Verfahren (DIW).

7. Ein Verfahren zur Herstellung eines intelligenten Verbundtextils, wobei das Verfahren umfasst:
das Erfassen einer dreidimensionalen räumlichen Verteilung von Poren eines biologischen Materials von Interesse;
Entwerfen eines additiven Fertigungsmusters auf der Grundlage der dreidimensionalen räumlichen Verteilung der Poren des biologischen Materials von Interesse;
das Zusammenfügen einer Vielzahl von Fasern auf der Grundlage eines Faseranordnungsmusters zu einem Textilsubstrat;
das Aufbringen eines Materials mittels eines additiven Fertigungsverfahrens auf und/oder zwischen Fasern des Textilsubstrats auf der Grundlage des additiven Fertigungsmusters, um ein Verbundtextil bereitzustellen, das einen Gradienten in mindestens einer der folgenden Eigenschaften aufweist:
mechanische Eigenschaft, Materialeigenschaft oder strukturelle Eigenschaft, und/oder das als Reaktion auf mindestens einen externen Reiz eine Veränderung in mindestens einer mechanischen Eigenschaft, Materialeigenschaft oder Struktur zeigt;
wobei das additive Fertigungsmuster auf einer dreidimensionalen räumlichen Verteilung von Poren in einem biologischen Material von Interesse basiert und das aufgebrachte Material eine Matrix definiert, die eine Vielzahl von Poren mit einer hierarchischen Porosität und/oder einem Porositätsmuster im Verbundtextil enthält.

8. Das Verfahren nach Anspruch 7, ferner umfassend:
das Abbilden einer dreidimensionalen räumlichen Verteilung der Struktur des biologischen Materials von Interesse;
Entwerfen des Faseranordnungsmusters auf der Grundlage der dreidimensionalen räumlichen Verteilung mindestens eines Strukturmoleküls des biologischen Materials von Interesse; und
das Zusammenfügen von Fasern auf der Grundlage des Faseranordnungsmusters, um das Textilsubstrat zu bilden.

9. Das Verfahren nach Anspruch 8, wobei das Strukturmolekül mindestens eine Strukturproteinfaser der extrazellulären Matrix umfasst.

10. Das Verfahren nach Anspruch 9, wobei die mindestens eine Strukturproteinfaser Kollagenfasern und Elastinfasern der extrazellulären Matrix des biologischen Materials von Interesse umfasst.

11. Das Verfahren nach Anspruch 10, wobei das Faseranordnungsmuster einen Webalgorithmus umfasst, der auf der dreidimensionalen räumlichen Verteilung der mindestens einen Strukturproteinfaser basiert, wobei die angeordneten Fasern unter Verwendung des Webalgorithmus vorzugsweise verwoben werden, um das Webmuster und die Faserausrichtung zu definieren.

12. Das Verfahren nach Anspruch 7, wobei das additive Fertigungsverfahren eine oder mehrere der folgenden Techniken umfasst: ein Fused-Deposition-Modeling-Verfahren (FDM), ein Fused-Filament-Fabrication-Verfahren (FFF), ein Big-Area-Additive-Manufacturing-Verfahren (BAAM), ein Robocasting-Verfahren, ein Pastenextrusionsverfahren und/oder ein Direct-Ink-Writing-Verfahren (DIW).

13. Das Verfahren nach Anspruch 7, wobei das additive Fertigungsmuster einen Druckalgorithmus umfasst, der auf der dreidimensionalen räumlichen Verteilung von Poren basiert.

## Revendications

1. Matériau intelligent comprenant :
un textile composite qui comporte un substrat textile formé à partir d'une pluralité de fibres assemblées selon un motif d'assemblage de fibres ; et
un matériau déposé par le biais d'une technique de fabrication additive sur et/ou entre les fibres du substrat textile sur la base d'un motif de fabrication additive, dans lequel le textile composite comporte un gradient dans au moins une parmi une propriété mécanique, une propriété de matériau ou une propriété structurale et/ou présente une modification d'au moins une propriété mécanique, une propriété de matériau ou une structure en réponse à au moins un stimulus externe,
dans lequel le motif de fabrication additive est basé sur une distribution spatiale tridimensionnelle de pores dans un matériau biologique d'intérêt et le matériau déposé définit une matrice qui comporte une pluralité de pores avec un motif de porosité et/ou de porosité hiérarchique dans le textile composite.

2. Matériau intelligent selon la revendication 1, dans lequel le motif d'assemblage de fibres est basé sur une distribution spatiale tridimensionnelle d'au moins une molécule structurale du matériau biologique d'intérêt ; et les fibres sont assemblées sur la base du motif d'assemblage de fibres pour former le substrat textile.

3. Matériau intelligent selon la revendication 2, dans lequel la molécule structurale comprend au moins une fibre de protéine structurale de la matrice extracellulaire.

4. Matériau intelligent selon la revendication 3, dans lequel l'au moins une fibre de protéine structurale comprend des fibres de collagène et des fibres d'élastine de la matrice extracellulaire du matériau biologique d'intérêt, dans lequel les fibres assemblées sont de préférence tissées à l'aide d'un algorithme de tissage basé sur la distribution spatiale tridimensionnelle de l'au moins une fibre de protéine structurale pour définir le motif de tissage et l'orientation des fibres.

5. Matériau intelligent selon la revendication 1, dans lequel le matériau biologique d'intérêt comprend un tissu d'une plante ou d'un animal.

6. Matériau intelligent selon la revendication 1, dans lequel la technique de fabrication additive comprend une ou plusieurs parmi une technique de dépôt de filament fondu (FDM), une technique de fabrication de filament fondu (FFF), une technique de fabrication additive grand format (BAAM), une technique de robocasting, une technique d'extrusion de pâte, une technique d'électrofilage et/ou une technique d'écriture directe à l'encre (DIW).

7. Procédé de formation d'un textile composite intelligent, le procédé comprenant :
la cartographie d'une distribution spatiale tridimensionnelle de pores d'un matériau biologique d'intérêt ;
la conception d'un motif de fabrication additive sur la base de la distribution spatiale tridimensionnelle de pores du matériau biologique d'intérêt ;
l'assemblage d'une pluralité de fibres sur la base d'un motif d'assemblage de fibres dans un substrat textile ;
le dépôt d'un matériau via une technique de fabrication additive sur et/ou entre des fibres du substrat textile sur la base du motif de fabrication additive pour fournir un textile composite qui comporte un gradient dans au moins une parmi une propriété mécanique, une propriété de matériau ou une propriété structurale et/ou qui présente une modification d'au moins une propriété mécanique, une propriété de matériau ou une structure en réponse à au moins un stimulus externe ;
dans lequel le motif de fabrication additive est basé sur une distribution spatiale tridimensionnelle de pores dans un matériau biologique d'intérêt et le matériau déposé définit une matrice qui comporte une pluralité de pores avec un motif de porosité et/ou de porosité hiérarchique dans le textile composite.

8. Procédé selon la revendication 7, comprenant en outre :
la cartographie d'une distribution spatiale tridimensionnelle de la structure du matériau biologique d'intérêt ;
la conception du motif d'assemblage de fibres sur la base de la distribution spatiale tridimensionnelle d'au moins une molécule structurale du matériau biologique d'intérêt ; et
l'assemblage de fibres sur la base du motif d'assemblage de fibres pour former le substrat textile.

9. Procédé selon la revendication 8, dans lequel la molécule structurale comprend au moins une fibre de protéine structurale de la matrice extracellulaire.

10. Procédé selon la revendication 9, dans lequel l'au moins une fibre de protéine structurale comprend des fibres de collagène et des fibres d'élastine de la matrice extracellulaire du matériau biologique d'intérêt.

11. Procédé selon la revendication 10, dans lequel le motif d'assemblage de fibres comprend un algorithme de tissage basé sur la distribution spatiale tridimensionnelle de l'au moins une fibre de protéine structurale, dans lequel les fibres assemblées sont de préférence tissées à l'aide de l'algorithme de tissage pour définir le motif de tissage et l'orientation des fibres.

12. Procédé selon la revendication 7, dans lequel la technique de fabrication additive comprend une ou plusieurs parmi une technique de dépôt de filament fondu (FDM), une technique de fabrication de filament fondu (FFF), une technique de fabrication additive grand format (BAAM), une technique de robocasting, une technique d'extrusion de pâte et/ou une technique d'écriture directe à l'encre (DIW).

13. Procédé selon la revendication 7, dans lequel le motif de fabrication additive comporte un algorithme d'impression basé sur la distribution spatiale tridimensionnelle de pores.
